(19) Europäisches Patentamt
European Patent Office
Office européen des brevets

(11) Veröffentlichungsnummer: **0 121 171**
**B1**

# (12) EUROPÄISCHE PATENTSCHRIFT

(45) Veröffentlichungstag der Patentschrift: **10.05.89**

(51) Int. Cl.⁴: **A 01 N 43/64,** A 01 N 43/50, A 01 N 43/56, A 01 N 39/00 // C07D249/08, C07D233/60, C07D231/12, C07D403/06, C07D405/06, C07D303/36, C07D303/34, C07D303/24, C07D303/22, C07C149/00, C07C43/00

(21) Anmeldenummer: **84102994.5**

(22) Anmeldetag: **19.03.84**

## (54) Fungizide Mittel, Verfahren zu ihrer Herstellung und deren Verwendung.

(30) Priorität: **30.03.83 DE 3311702**

(43) Veröffentlichungstag der Anmeldung: **10.10.84 Patentblatt 84/41**

(45) Bekanntmachung des Hinweises auf die Patenterteilung: **10.05.89 Patentblatt 89/19**

(84) Benannte Vertragsstaaten: **AT BE CH DE FR GB IT LI NL SE**

(56) Entgegenhaltungen:
**EP-A-0 043 419**
**EP-A-0 044 605**
**EP-A-0 087 148**
**EP-A-0 105 166**

(73) Patentinhaber: **BAYER AG, Konzernverwaltung RP Patentabteilung, D-5090 Leverkusen 1 Bayerwerk (DE)**

(72) Erfinder: **Regel, Erik, Dipl.- Ing., Untere Bergerheide 26, D-5600 Wuppertal 1 (DE)**
Erfinder: **Büchel, Karl Heinz, Prof. Dr., Dabringhausener Strasse 42, D-5093 Burscheid (DE)**
Erfinder: **Reinecke, Paul, Dr., Lessingstrasse 11, D-5090 Leverkusen 3 (DE)**
Erfinder: **Brandes, Wilhelm, Dr., Eichendorffstrasse 3, D-5653 Leichlingen 1 (DE)**

EP 0 121 171 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft die Verwendung von substituierten tert.-Butanol-Derivaten, die in der nicht vorveroffentlichten EP-A-0 105 166 beschrieben werden, als Fungizide.

Es ist bereits bekannt geworden, daß bestimmte Diazolyl-Derivate, wie beispielsweise 2-(4-Chlorphenyl)- oder -(2-Chlorphenyl)-1,3-di(1,2,4-triazol-1-yl)-2-propanol bzw. 2-(4-Chlorphenyl)-1,3-di(imidazol-1-yl)-2-propanol, gute fungizide Eigenschaften aufweisen (vergleiche EP-A-0 044 605). Die Wirkung dieser Verbindungen ist jedoch, insbesondere bei niedrigen Aufwandmengen und -konzentrationen, nicht immer ganz befriedigend.

Es wurde nun gefunden, daß die substituierten tert.-Butanol-Derivate der Formel

$$\begin{array}{c} OH \\ | \\ R^1 - CH_2 - C - CH_2 - R^2 \\ | \\ CH_2 \\ | \\ R^3 \end{array} \qquad (I)$$

in welcher

$R^1$, $R^2$ und $R^3$ gleich oder verschieden sind und für Azolyl, gegebenenfalls substituiertes Phenoxy, gegebenenfalls substituiertes Phenylthio, Alkylthio, Alkenyl, Alkinyl, gegebenenfalls substituiertes Phenylacetylenyl, Alkylamino, Dialkylamino, gegebenenfalls substituiertes Phenylamino, gegebenenfalls substituiertes Phenyl-N-alkyl-amino, gegebenenfalls substituiertes Cycloalkylamino, gegebenenfalls substituiertes Cycloalkyl-N-alkyl-amino, Aminoethoxy, Alkylaminoethoxy oder Dialkylaminoethoxy stehen,

sowie deren Säureadditionssalze und Metallsalzkomplexe gute fungizide Eigenschaften aufweisen.

Überraschenderweise zeigen die erfindungsgemäß zu verwendenden substituierten tert.-Butanol-Derivate der Formel (I) eine bessere fungizide Wirksamkeit als die aus dem Stand der Technik bereits bekannten Diazolyl-Derivate 2-(4-Chlorphenyl)- oder -(2-Chlorphenyl)-1,3-di(1,2,4-triazol-1-yl)-2-propanol und 2-(4-Chlorphenyl)-1,3-di(imidazol-1-yl)-2-propanol, welche konstitutionell und wirkungsmäßig naheliegende Verbindungen sind. Die erfindungsgemäße Verwendung der neuen Stoffe stellt somit eine Bereicherung der Technik dar.

Die erfindungsgemäß zu verwendenden substituierten tert.-Butanol-Derivate sind durch die Formel (I) allgemein definiert.

Bevorzugt sind Verbindungen der Formel (I), in denen

A) $R^1$ und $R^2$ gleich oder verschieden sind und für Imidazol-1-yl, 1,2,4-Triazol-1-yl, 1,2,4-Triazol-4-yl oder Pyrazol-1-yl stehen und

$R^3$ für jeweils gegebenenfalls einfach oder mehrfach, gleich oder verschieden substituiertes Phenoxy, Phenylthio, Phenylamino oder Phenyl-N-alkyl-amino mit 1 bis 4 Kohlenstoffatomen im Alkylteil steht, wobei die genannten Gruppen im Phenylteil substituiert sein können durch Halogen, Alkyl mit 1 bis 6 Kohlenstoffatomen, Alkoxy und Alkylthio mit jeweils 1 bis 4 Kohlenstoffatomen; durch Halogenalkyl, Halogenalkoxy und Halogenalkylthio mit jeweils 1 bis 4 Kohlenstoffatomen und 1 bis 5 gleichen oder verschiedenen Halogenatomen, wie Fluor- und Chloratomen, Cycloalkyl mit 5 bis 6 Kohlenstoffatomen; ferner durch Nitro, Cyano, Hydroxycarbonyl, Alkylcarbonyl oder Alkoxycarbonyl mit jeweils 1 bis 4 Kohlenstoffatomen im Alkylteil, gegebenenfalls einfach oder mehrfach, gleich oder verschieden durch Halogen, Nitro, Trifluormethyl und Alkyl mit 1 bis 2 Kohlenstoffatomen substituiertes Phenyl oder Phenoxy, durch die Aldehydgruppe sowie den Oxim- bzw. Oximether-Rest.

Bevorzugt sind außerdem Verbindungen der allgemeinen Formel (I), in denen

B) $R^1$ und $R^2$ gleich oder verschieden sind und für Imidazol-1-yl; 1,2,4-Triazol-1-yl; 1,2,4-Triazol-4-yl oder Pyrazol-1-yl stehen und

$R^3$ für Alkylthio mit 1 bis 12 Kohlenstoffatomen, für Alkylamino oder Dialkylamino mit jeweils 1 bis 4 Kohlenstoffatomen in jedem Alkylteil, für Aminoethoxy, Alkylaminoethoxy und Dialkylaminoethoxy mit jeweils 1 bis 4 Kohlenstoffatomen in jedem Alkylteil, sowie für jeweils gegebenenfalls im Cycloalkylteil einfach oder mehrfach gleich oder verschieden substituiertes Cycloalkylamino und Cycloalkyl-N-alkylamino mit jeweils 3 bis 7 Kohlenstoffatomen im Cycloalkylteil und 1 bis 4 Kohlenstoffatomen im Alkylteil steht, wobei als Substituenten infrage kommen: Halogen, Alkyl mit 1 bis 4 Kohlenstoffatomen und Alkoxy mit 1 bis 2 Kohlenstoffatomen.

Bevorzugt sind ferner Verbindungen der Formel (I), in denen

| | |
|---|---|
| C) $R^1$ | für Imidazol-1-yl; 1,2,4-Triazol-1-yl; 1,2,4-Triazol-4-yl oder Pyrazol-1-yl steht und |
| $R^2$ und $R^3$ | gleich oder verschieden sind und für die oben unter Punkt A) genannten Substituenten für $R^3$ stehen. |

Bevorzugt sind weiterhin Verbindungen der Formel (I), in denen

| | |
|---|---|
| D) $R^1$ | für Imidazol-1-yl; 1,2,4-Triazol-1-yl; 1,2,4-Triazol-4-yl oder Pyrazol-1-yl steht und |
| $R^2$ und $R^3$ | gleich oder verschieden sind und für die oben unter Punkt B) genannten Bedeutungen für $R^3$ stehen. |

Bevorzugt sind ebenfalls Verbindungen der Formel (I), in denen

| | |
|---|---|
| E) $R^1$, $R^2$ und $R^3$ | gleich oder verschieden sind und für die oben unter den Punkten A) und B) genannten Bedeutungen für $R^3$ stehen. |

Bevorzugt sind schließlich auch Verbindungen der Formel (I), in denen

| | |
|---|---|
| F) $R^1$, $R^2$ und $R^3$ | gleich oder verschieden sind und für die oben unter Punkt A) genannten Bedeutungen für $R^1$ stehen. |

Besonders bevorzugt sind diejenigen Verbindungen der Formel (I), in denen

| | |
|---|---|
| A) $R^1$ und $R^2$ | gleich oder verschieden sind und für Imidazol-1-yl; 1,2,4-Triazol-1-yl; 1,2,4-Triazol-4-yl oder Pyrazol-1-yl stehen und |
| $R^3$ | für jeweils gegebenenfalls einfach bis dreifach, gleich oder verschieden substituiertes Phenoxy, Phenylthio, Phenylamino oder Phenyl-N-alkyl-amino mit 1 bis 2 Kohlenstoffatomen im Alkylteil steht, wobei als Phenylsubstituenten jeweils infrage kommen: Fluor, Chlor, Brom, Methyl, Ethyl, Isopropyl, tert.-Butyl, 2-Methyl-but-2-yl, Methoxy, Ethoxy, Methylthio, Trifluormethyl, Trifluormethoxy, Trifluormethylthio, Cyclohexyl, Nitro, Cyano, Hydroxycarbonyl, Methylcarbonyl, Methoxycarbonyl, Ethoxycarbonyl, jeweils gegebenenfalls einfach bis dreifach, gleich oder verschieden durch Chlor, Nitro, Trifluormethyl oder Methyl substituiertes Phenyl oder Phenoxy, die Aldehydgruppe, Hydroxyiminomethyl und Methoximinomethyl. |

Besonders bevorzugt sind außerdem diejenigen Verbindungen der Formel (I) in denen

| | |
|---|---|
| B) $R^1$ und $R^2$ | gleich oder verschieden sind und für Imidazol-1-yl; 1,2,4-Triazol-1-yl; 1,2,4-Triazol-4-yl oder Pyrazol-1-yl stehen und |
| $R^3$ | für Methylthio, Ethylthio, Butylthio und Dodecylthio steht, ferner für Alkylamino und Dialkylamino mit jeweils 1 bis 2 Kohlenstoffatomen in jedem Alkylteil steht, ferner für Aminoethoxy, Alkylaminoethoxy und Dialkylaminoethoxy mit jeweils 1 bis 2 Kohlenstoffatomen in jedem Alkylteil steht sowie für jeweils gegebenenfalls im Cycloalkylteil einfach bis dreifach, gleich oder verschieden substituiertes Cyclopropylamino, Cyclopentylamino, Cyclohexylamino, Cyclopropyl-N-methyl-amino, Cyclopentyl-N-methyl-amino und Cyclohexyl-N-methylamino steht, wobei als Substituenten infrage kommen: Chlor, Brom, Methyl, Ethyl, Isopropyl und Methoxy. |

Besonders bevorzugt sind ferner diejenigen Verbindungen der Formel (I), in denen

| | |
|---|---|
| C) $R^1$ | für Imidazol-1-yl; 1,2,4-Triazol-1-yl; 1,2,4-Triazol-4-yl oder Pyrazol-1-yl steht und |
| $R^2$ und $R^3$ | gleich oder verschieden sind und für die oben unter Punkt A) genannten Bedeutungen für $R^3$ stehen. |

Besonders bevorzugt sind weiterhin diejenigen Verbindungen der Formel (I), in denen

| | |
|---|---|
| D) $R^1$ | für Imidazol-1-yl; 1,2,4-Triazol-1-yl; 1,2,4-Triazol-4-yl oder Pyrazol-1-yl steht und |
| $R^2$ und $R^3$ | gleich oder verschieden sind und für die oben unter Punkt B) genannten Bedeutungen für $R^3$ stehen. |

Besonders bevorzugt sind ebenfalls diejenigen Verbindungen der Formel (I), in denen

| | |
|---|---|
| E) $R^1$, $R^2$ und $R^3$ | gleich oder verschieden sind und für die oben unter den Punkten A) und B) genannten Bedeutungen für $R^3$ stehen. |

Besonders bevorzugt sind schließlich auch diejenigen Verbindungen der Formel (I), in denen

F) $R^1$, $R^2$ und $R^3$ gleich oder verschieden sind und für die oben unter Punkt A) genannten Bedeutungen für $R^1$ stehen.

Bevorzugte erfindungsgemäße Verbindungen sind auch Additionsprodukte aus Säuren und denjenigen substituierten tert.-Butanol-Derivaten der Formel (I), in denen die Substituenten $R^1$, $R^2$ und $R^3$ die Bedeutungen haben, die bereits vorzugsweise für diese Substituenten genannt wurden.

Zu den Säuren die addiert werden können, gehören vorzugsweise Halogenwasserstoffsäuren, wie z. B. Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure und Milchsäure, sowie Sulfonsäuren, wie p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen substituierten tert.-Butanol-Derivaten der Formel (I), in denen die Substituenten $R^1$, $R^2$ und $R^3$ die Bedeutungen haben, die bereits vorzugsweise für diese Substituenten genannt wurden.

Hierbei sind die Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z. B. die Chlorwasserstoffsäure und die Bromwasserstoffsäure, Salpetersäure und Schwefelsäure.

Im einzelnen seien außer den bei den Herstellungsbeispielen genannten Verbindungen die folgenden Verbindungen der allgemeinen Formel (I) genannt:

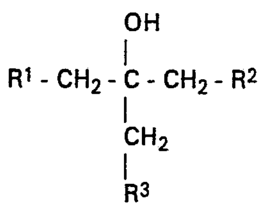

(I)

| $R^1$ | $R^2$ | $R^3$ |
|---|---|---|
| 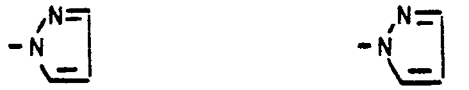 | | 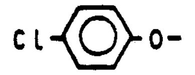 |
| 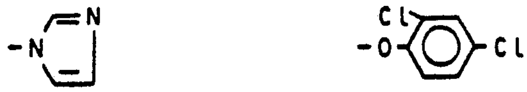 | | 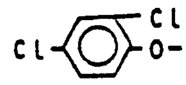 |
| 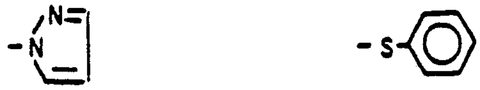 | | 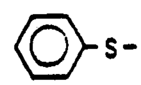 |
| 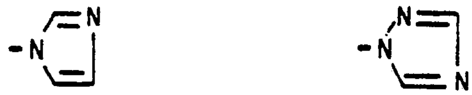 | | 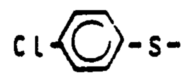 |
| 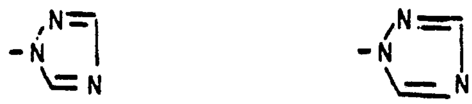 | | 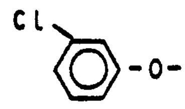 |
| 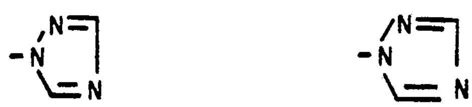 | | 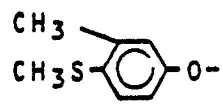 |
| 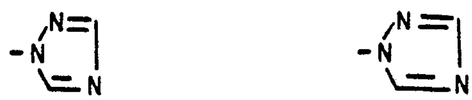 | | 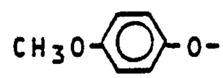 |
| 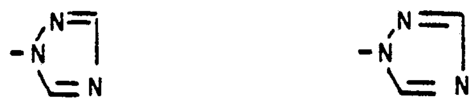 | | 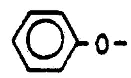 |
| 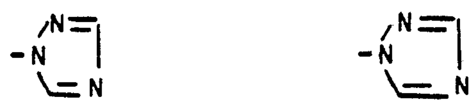 | | 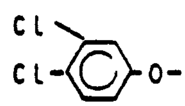 |
| 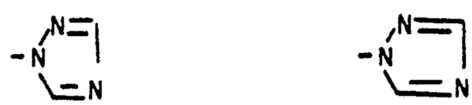 | | $(C_2H_5)_2N-CH_2CH_2-O-$ |
| 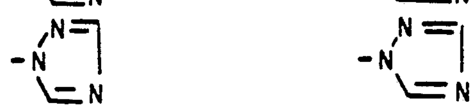 | | 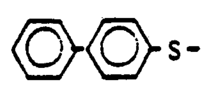 |
| 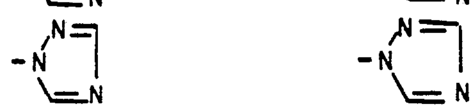 | | 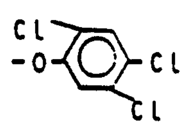 |
| 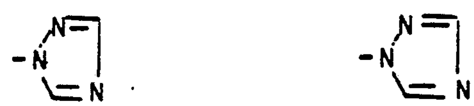 | | 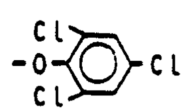 |
| 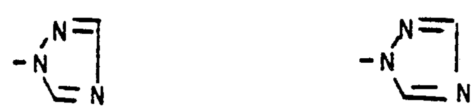 | | 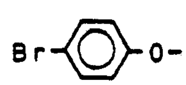 |
| 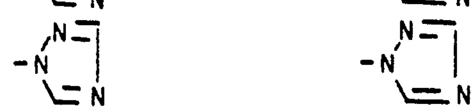 | | 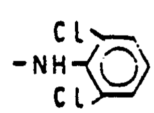 |

Die erfindungsgemäß in verwendenden Stoffe werden in der vorgängigen, aber nicht vorveröffentlichten EP-A-0 105 166 beschrieben. Diese Wirkstoffe werden erhalten, wenn man ein Nucleophil der Formel

$R^1$ - H (II)

in welcher
$R^1$ die oben angegebene Bedeutung hat,

a) mit 2,2-Dihalogenmethyloxiranen der Formel

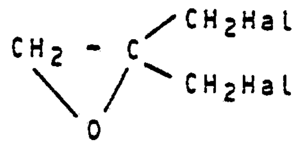 (III)

in welcher
Hal für Halogen steht,

in Gegenwart eines inerten organischen Lösungsmittels, wie beispielsweise Aceton, und in Gegenwart eines Säurebindemittels, wie beispielsweise Kaliumcarbonat, bei Temperaturen zwischen 20 und 120°C umsetzt, oder

b) mit 2-Halogenmethyloxiranen der Formel

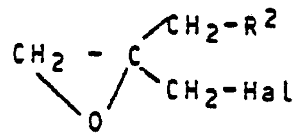 (IV)

in welcher
$R^2$ und Hal die oben angegebene Bedeutung haben,

in Gegenwart eines inerten organischen Lösungsmittels, wie beispielsweise Aceton, und in Gegenwart eines Säurebindemittels, wie beispielsweise Kaliumcarbonat, bei Temperaturen zwischen 20 und 120°C umsetzt, oder

c) mit substituierten Oxiranen der Formel

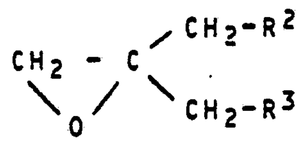 (V)

in welcher
$R^2$ und $R^3$ die oben angegebene Bedeutung haben,

in Gegenwart eines inerten organischen Lösungsmittels, wie beispielsweise Aceton, und in Gegenwart eines Säurebindemittels, wie beispielsweise Kaliumcarbonat, bei Temperaturen zwischen 20 und 120°C umsetzt.

Die Nucleophile der Formel (II) sind allgemein bekannte Verbindungen der organischen Chemie.

Die 2,2-Dihalogenmethyloxirane der Formel (III) sind bekannt (vgl. Beilstein, E III (1), S. 1587 - 1588), bzw. können sie in bekannter Art und Weise erhalten werden, indem man 3-Halogen-2-halogenmethylpropene der Formel

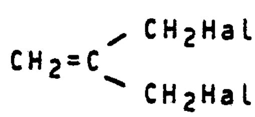 (VI).

in welcher
Hal die oben angegebene Bedeutung hat,

entweder zunaächst mit tert.-Butyloxy-halogenid zu 1,3-Dihalogen-2-halogenmethyl-2-propanolen der Formel

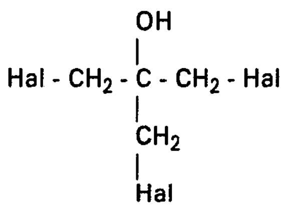

(VII)

in welcher
Hal die oben angegebene Bedeutung hat,

umsetzt und diese anschließend in Gegenwart von Calciumhydroxid zu den gewünschten 2,2-Dihalogenmethyloxiranen der Formel (III) epoxidiert (vgl. hierzu auch Beilstein, E III (1), S. 1587 - 1588);

oder direkt in Gegenwart von Persäuren, wie z. B. Peressigsäure oder m-Chlorperbenzoesäure, zu den gewünschten 2,2-Dihalogenmethyloxiranen der Formel (III) epoxidiert.

Die 2-Halogenmethyloxirane der Formel (IV) sind noch nicht bekannt. Sie können jedoch in allgemein üblicher Art und Weise erhalten werden, indem man

entweder 2,2-Dihalogenmethyloxirane der Formel (III) mit einem Nucleophil der Formel (II) umsetzt;

oder 3-Halogen-2-halogenmethyl-propene der Formel (VI) zunächst mit einem Nucleophil der Formel (II), gegebenenfalls in Form eines Alkalimetallsalzes, zu Propenen der Formel

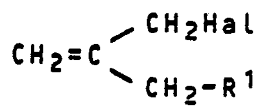

(VIII)

in welcher
Hal und $R^1$ die oben angegebene Bedeutung haben,

umsetzt und diese dann in Gegenwart von Persäuren zu den gewünschten 2-Halogenmethyloxiranen der Formel (IV) epoxidiert.

Die substituierten Oxirane der Formel (V) sind noch nicht bekannt. Sie können jedoch in allgemein üblicher Art und Weise erhalten werden, indem man

entweder 2,2-Dihalogenmethyloxirane der Formel (III) mit einem Nucleophil der Formel (II) umsetzt;

oder 3-Halogen-2-halogenmethyl-propene der Formel (VI) mit einem Nucleophil der Formel (II), gegebenenfalls in Form eines Alkalimetallsalzes, zu Propenen der Formel

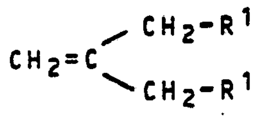

(IX)

in welcher
$R^1$ die oben angegebene Bedeutung hat,

umsetzt und diese dann in Gegenwart von Persäuren zu den gewünschten Oxiranen der Formel (V) epoxidiert;

oder Ketone der Formel

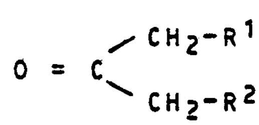

(X)

in welcher
$R^1$ und $R^2$ die oben angegebene Bedeutung haben,

in an sich bekannter Art und Weise mit Dimethylsulfoniummethylid epoxidiert.

Bestimmte Verbindungen der Formel (I) können auch erhalten werden, indem man Di(halogenmethyl)-carbinole der Formel (XI)

$$\begin{array}{c} CH_2Hal \\ | \\ R^4 - CH_2 - C - OH \\ | \\ CH_2Hal \end{array} \qquad (XI)$$

in welcher

Hal die oben angegebene Bedeutung hat und

$R^1$ für gegebenenfalls substituiertes Phenylthio, Alkylthio, Alkylamino, Dialkylamino, gegebenenfalls substituiertes Phenylamino, gegebenenfalls substituiertes Phenyl-N-alkylamino, gegebenenfalls substituiertes Cycloalkylamino oder gegebenenfalls substituiertes Cycloalkyl-N-alkyl-amino steht,

gemäß den Bedingungen des Verfahrens (a) umsetzt.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindunsgemäßen Wirkstoffe mit besonders gutem Erfolg zur Bekämpfung von Getreidekrankheiten, wie Erysiphe graminis, Leptosphaeria nodorum, Puccinia sp., Cochliobolus sativus und Pyrenophora teres; von Sphaerotheca-Arten, wie Sphaerotheca fuliginea sowie von Reiskrankheiten, wie Pyricularia und Pellicularia eingesetzt werden. Darüber hinaus weisen die erfindungsgemäßen Wirkstoffe ein breites fungizides in-vitro-Wirkungsspektrum auf.

In entsprechenden Aufwandmengen zeigen die erfindungsgemäßen Verbindungen auch pflanzenwachstumsregulierende Wirkung.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Ärosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä. sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden.

Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, starke polare Lösungsmittel, wie Dimethylformamid und Dimethylsulfoxid, sowie Wasser;

mit verflüssigten gasförmigen Streckmitteln oder Trägerstoffen sind solche Flüssigkeiten gemeint, welche bei normaler Temperatur und unter Normaldruck gasförmig sind, z. B. Ärosol-Treibgase, wie Halogenkohlenwasserstoffe sowie Butan, Propan, Stickstoff und Kohlendioxid;

als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate;

als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln;

als Emulgier- und/oder schaumerzeugende Mittel kommen in Frage: z. B. nichtionogene und anionische Emulgatoren, wie Polyoxyethylen-Fettsäureester, Polyoxyethylen-Fettalkohol-ether, z. B. Alkylarylpolyglykolether, Alkylsulfonate, Alkylsulfate, Arylsulfonate sowie Eiweißhydrolysate;

als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- u. Metallphthalocyaninfarbstoffe und Spurennährstoffe wie Salze von Eisen,

Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-% am Wirkungsort erforderlich.

**Herstellungsbeispiele:**

**Beispiel 1 und 2**

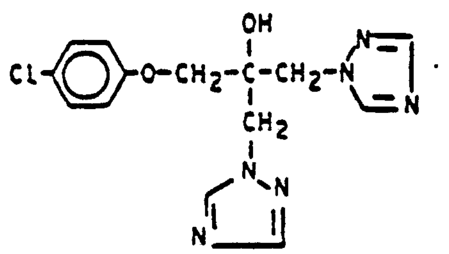

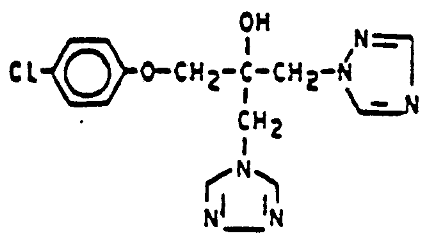

(Beispiel 1) (Beispiel 2)

(Verfahren B)

Zu einem Gemisch von 13,6 g (0,2 Mol) 1,2,4-Triazol und 13,8 g (0,1 Mol) Kaliumcarbonat in 200 ml Aceton werden unter Rühren 9,4 g (0,04 Mol) 2-Chlormethyl-2-(4-chlorphenoxymethyl)-oxiran getropft. Man läßt 15 Stunden bei Raumtemperatur und anschließend 22 Stunden unter Rückfluß rühren. Das Reaktionsgemisch wird danach kalt filtriert und das Filtrat im Vakuum eingeengt. Der ölige Rückstand wird in Chloroform gelöst, mit Wasser gewaschen, über Natriumsulfat getrocknet und chromatographisch (Kieselgel 60 Merck, Chloroform/Methanol = 20/1) gereinigt. Man erhält 5,8 g (43 % der Theorie) 2-(4-Chlorphenoxymethyl)-1,3-di(1,2,4-triazol-1-yl)-2-hydroxypropan vom Schmelzpunkt 99°C (Beispiel 1) und 2,0 g (15 % der Theorie) 2-(4-Chlorphenoxymethyl)-2-hydroxy-1-(1,2,4-triazol-1-yl)-3-(1,2,4-triazol-4-yl)-propan (Beispiel 2) vom Schmelzpunkt 160°C.

**Herstellung des Ausgangsproduktes**

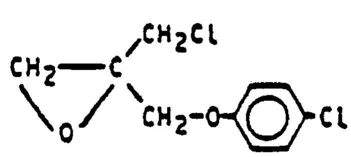

(IV - 1)

(1. Variante)

12,85 g (0,1 Mol) 4-Chlorphenol in 50 ml Aceton werden zu einem Gemisch von 14,1 g (0,1 Mol) 2,2-Di(chlormethyl)-oxiran, 13,8 g (0,1 Mol) Kaliumcarbonat in 200 ml Aceton getropft. Man erhitzt 18 Stunden unter Rückfluß, läßt abkühlen und filtriert. Das Filtrat wird im Vakuum eingeengt, der Rückstand in Chloroform gelöst, mit Wasser gewaschen, über Natriumsulfat getrocknet und eingeengt.

Nach destillativer Reinigung erhält man 7,6 g (32,5 % der Theorie) 2-Chlormethyl-2-(4-chlorphenoxymethyl)-oxiran vom Siedepunkt 150°C/0,5 mbar.

(2. Variante)

0,5 Mol 4-Chlorphenol-natrium in Acetonitril werden zu einer Lösung von 125 g (1 Mol) 3-Chlor-2-chlormethylpropen in 50 ml Acetonitril getropft. Nach Zusatz von 0,5 g Natriumjodid wird das Reaktionsgemisch 12 Stunden unter Rückfluß erhitzt und anschließend kalt filtriert. Das Filtrat wird im Vakuum eingeengt. Der Rückstand wird in Methylenchlorid gelöst, mit Wasser gewaschen, über Natriumsulfat getrocknet und eingeengt. Man erhält 58,6 g ca. 60 %-iges 2-Chlormethyl-3-(4-chlorphenoxy)-propen, was in 500 ml Methylenchlorid gelöst und mit 43 g (0,25 Mol) 3-Chlorperbenzoesäure versetzt wird.

Man läßt 24 Stunden bei Raumtemperatur rühren und filtriert. Das filtrat wird mit wässriger, 10 %-iger Natriumthiosulfatlösung gewaschen, über Natriumsulfat getrocknet und im Vakuum eingeengt.

Nach destillativer Reinigung erhält man 30 g (12,9 % der Theorie) 2-Chlormethyl-2-(4-chlorphenoxymethyl)-oxiran vom Brechungsindex $n^{20}_D = 1,5465$.

**Beispiel 3, 4 und 5**

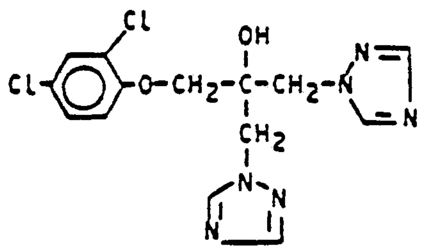

(Beispiel 3)

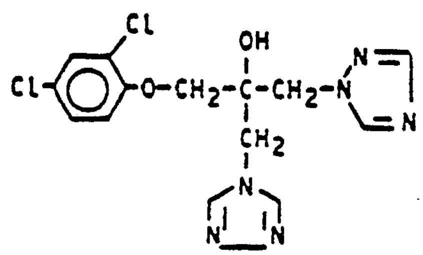

(Beispiel 4)

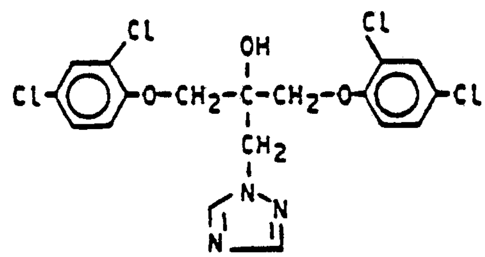

(Beispiel 5)

(Verfahren b und c)

23,5 g eines Gemisches aus 2-Chlormethyl-2-(2,4-dichlorphenoxymethyl)-oxiran und 2,2-Bis(2,4-dichlorphenoxymethyl)-oxiran werden zu einer Lösung von 20,7 g (0,3 Mol) 1,2,4-Triazol und 13,8 g (0,1 Mol) Kaliumcarbonat in 200 ml Aceton getropft. Man läßt das Reaktionsgemisch 20 Stunden unter Rückfluß rühren. Danach wird kalt filtriert und das Filtrat im Vakuum eingeengt. Der Rückstand wird in Chloroform gelöst, mit Wasser gewaschen, über Natriumsulfat getrocknet und chromatographisch (Kieselgel 60 Merck) gereinigt und getrennt. Zuerst wird mit Chloroform eluiert. Das dabei erhaltene Eluat wird durch Abdestillieren des Lösungsmittels eingeengt und der Rückstand mit Diethylether verrührt. Man erhält 4,8 g 1,3-Bis(2,4-dichlorphenoxy)-2-hydroxy-2-(1,2,4-triazol-1-yl-methyl)-propan (Beispiel 5) vom Schmelzpunkt 136° C.

Anschließend wird mit Chloroform/Methanol : 40/1 eluiert. Das dabei erhaltene Eluat wird durch Abdestillieren des Lösungsmittels eingeengt und der Rückstand mit Acetonitril verrührt. Man erhält 3,9 g 2-(2,4-Dichlorphenoxymethyl)-1,3-di(1,2,4-triazol-1-yl)-2-hydroxy-propan (Beispiel 3) vom Schmelzpunkt 138° C.

Zum Schluß wird mit Chloroform/Methanol : 20/1 eluiert. Das dabei erhaltene Eluat wird durch Abdestillieren des Lösungsmittels eingeengt und der Rückstand mit Acetonitril verrührt. Man erhalt 3,7 g 2-(2,4-Dichlorphenoxymethyl)-2-hydroxy-1-(1,2,4-triazol-1-yl)-3-(1,2,4-triazol-4-yl)-propan (Beispiel 4) vom Schmelzpunkt 182° C.

**Herstellung der Ausgangsprodukte**

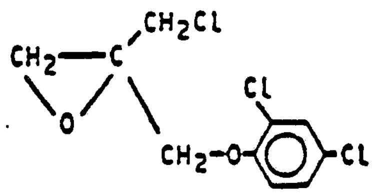

(IV - 2)

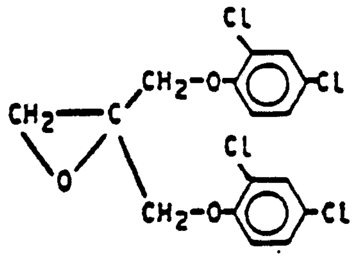

(V - 1)

Ein Gemisch von 14,1 g (0,1 Mol) 2,2-Di(chlormethyl)-oxiran, 13,8 g (0,1 Mol) Kaliumcarbonat und 16,3 g (0,1 Mol) 2,4-Dichlorphenol in 150 ml Aceton wird 15 Stunden auf 60°C erhitzt, anschließend kalt filtriert und das Filtrat im Vakuum eingeengt. Der Rückstand wird in Methylenchlorid aufgenommen, mit Wasser gewaschen, über Natriumsulfat getrocknet und eingeengt. Man erhält 23,5 g eines öligen Gemisches, das aufgrund gaschromatographischer Ermittlung neben 14,9 % unumgesetztem 2,2-Di(chlormethyl)-oxiran 47 % 2-Chlormethyl-2-(2,4-dichlorphenoxymethyl)-oxiran (Beisp. IV-2) und 24,9 % 2,2-Bis(2,4-dichlorphenoxymethyl)-oxiran (Beisp. V-1) enthält.

Dieses Gemisch kann gegebebenfalls chromatographisch getrennt werden, wobei 2-Chlormethyl-2-(2,4-dichlorphenoxymethyl)-oxiran (Beisp. IV-2) mit einem Brechungsindex $n^{20}_D$ = 1,5568 und 2,2-Bis(2,4-dichlorphenoxymethyl)-oxiran (Beisp. V-1) vom Schmelzpunkt 64°C erhalten werden.

**Beispiel 6**

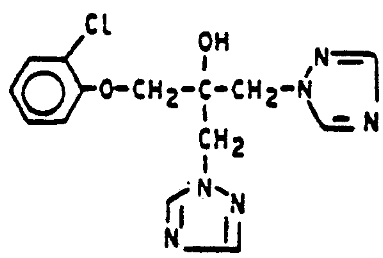

(Verfahren b)

Entsprechend Beispiel 1/2 erhält man aus 8,9 g (0,038 Mol) 2-Chlormethyl-2-(2-chlorphenoxymethyl)-oxiran, 5 g (0,038 Mol) Kaliumcarbonat und 6,9 g (0,1 Mol) 1,2,4-Triazol in 200 ml Aceton nach chromatographischer Reinigung des Reaktionsproduktes (Kieselgel 60, Merck, Chloroform/Methanol = 40/1) 7,1 g (55 % der Theorie) 2-(2-Chlorphenoxymethyl)-1,3-di(1,2,4-triazol-1-yl)-2-hydroxypropan vom Schmelzpunkt 95°C.

**Herstellung des Ausgangsproduktes**

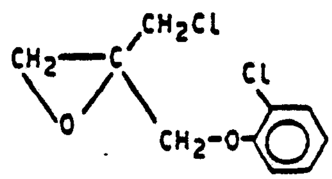

(IV - 3)

Entsprechend Beispiel 1/2 (Herstellung des Ausgangsproduktes, 1. Variante) erhält man aus 12,85 g (0,1 Mol) 2-Chlorphenol in 50 ml Aceton und 14,1 g (0,1 Mol) 2,2-Di(chlormethyl)-oxiran sowie 13,8 g (0,1 Mol) Kaliumcarbonat in 200 ml Aceton 9,1 g (39 % der Theorie) 2-Chlormethyl-2-(2-chlorphenoxymethyl)-oxiran vom Brechungindex $n^{20}_D$ = 1,5463.

**Beispiel 7**

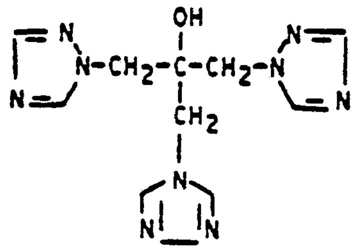

(Verfahren a)

28,2 g (0,2 Mol) 2,2-Di(chlormethyl)-oxiran werden unter Rühren zu einem Gemisch von 82,5 g (1,2 Mol) 1,2,4-Triazol und 82,5 g (0,6 Mol) Kaliumcarbonat in 400 ml Aceton getropft. Man läßt das Reaktionsgemisch 50 Stunden unter Rückfluß rühren, filtriert und engt das Filtrat im Vakuum ein. Der ölige Rückstand wird chromatographiert (Kieselgel 60, Merck, Chloroform/Methanol = 20/1). Man erhält 5,9 g (10,7 % der Theorie) 1,3-Di(1,2,4-triazol-1-yl)-2-hydroxy-2-(1,2,4-triazol-4-yl-methyl)-propan vom Schmelzpunkt 220° C.

**Beispiel 8 und 9**

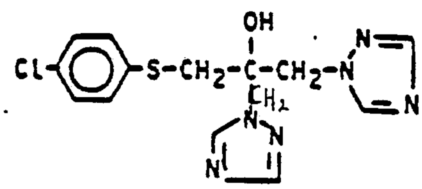

(Beispiel 8)

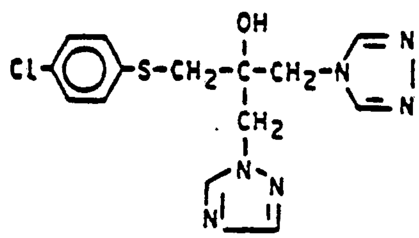

(Beispiel 9)

(Verfahren b)

Entsprechend Beispiel 1/2 erhält man aus 14,8 g (0,06 Mol) 2-Chlormethyl-2-(4-chlorphenylthiomethyl)-oxiran, 8,3 g (0,12 Mol) 1,2,4-Triazol und 8,3 g (0,06 Mol) Kaliumcarbonat in 200 ml Aceton 12,1 g (57,5 % der Theorie) 2-(4-Chlorphenylthiomethyl)-1,3-di(1,2,4-triazol-1-yl)-2-hydroxy-propan (Beispiel 8) vom Schmelzpunkt 150° C und 4,6 g (22 % der Theorie) 2-(4-Chlorphenylthiomethyl)-2-hydroxy-1-(1,2,4-triazol-1-yl)-3-(1,2,4-triazol-4-yl)-propan (Beispiel 9) vom Schmelzpunkt 186° C.

**Herstellung des Ausgangsproduktes**

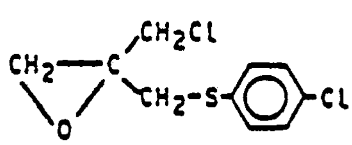

(IV - 4)

5,4 g (0,1 Mol) Natriummethylat werden portionsweist in ein Gemisch von 16,9 g (0,12 Mol) 2,2-Di(chlormethyl)-oxiran und 14,5 g (0,1 Mol) 4-Chlorthiophenol in 200 ml Acetonitril eingetragen. Man läßt das Reaktionsgemisch 4 Stunden nachrühren, filtriert und engt das Filtrat im Vakuum ein. Der Rückstand wird in Methylenchlorid gelöst, mit Wasser gewaschen, über Natriumsulfat getrocknet und eingeengt. Das verbleibende Öl wird destilliert. Man erhält 18,2 g (73 % der Theorie) 2-Chlormethyl-2-(4-chlorphenylthiomethyl)-oxiran vom Brechungsindex $n^{20}_D$ = 1,5895.

In entsprechender Weise und gemäß den erfindungsgemäßen Verfahren werden die folgenden Endprodukte der allgemeinen Formel (I)

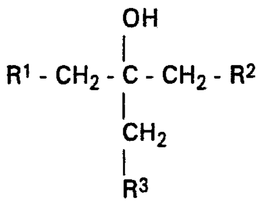 (I)

in Tabelle 1 erhalten:

| Beisp.-Nr. | $R^1$ | $R^2$ | $R^3$ | Schmp.(°C) oder $n_D^{20}$ |
|---|---|---|---|---|
| 10 | 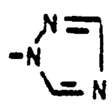 | 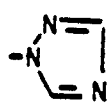 | 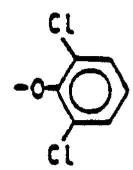 | 166 |
| 11 | " | " | 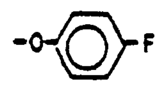 | 90 |
| 12 | " | " | 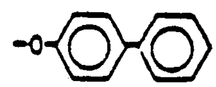 | 140 |
| 13 | " | " | 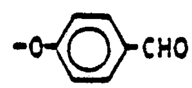 | 179 |
| 14 | " | " | 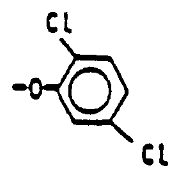 | 130 |
| 15 | " | 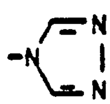 | 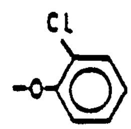 | 146 |
| 16 | " | " | 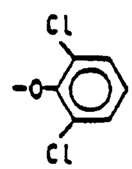 | 150 |
| 17 | " | " | 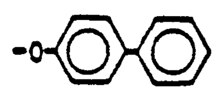 | 204 |
| 18 | " | " | 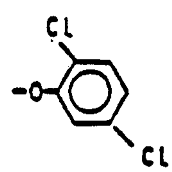 | 166 |

Tabelle 1 - Fortsetzung

| Beisp.-Nr. | $R^1$ | $R^2$ | $R^3$ | Schmp.(°C) oder $n_D^{20}$ |
|---|---|---|---|---|
| 19 | 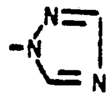 | 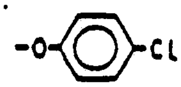 | 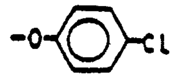 | 120 |
| 20 | 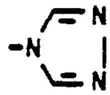 | 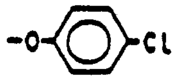 | 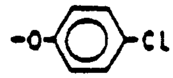 | 130 |
| 21 | 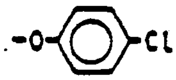 | 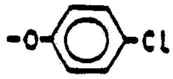 | 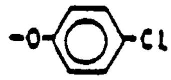 | 77 |
| 22 | 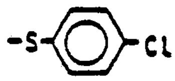 | 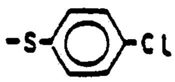 | 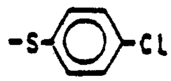 | 95 |
| 23 | 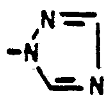 | 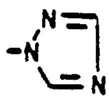 | 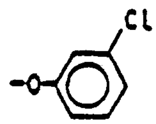 | 92 |
| 24 | 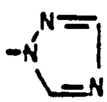 | 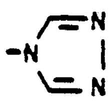 | 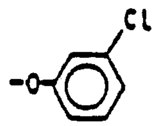 | 130 |
| 25 | 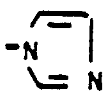 | 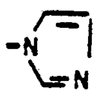 | 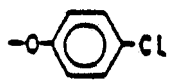 | 100 |
| 26 | 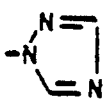 | 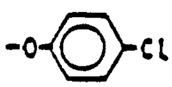 | 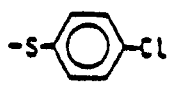 | 108 |
| 27 | 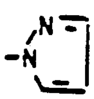 | 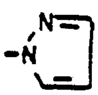 | 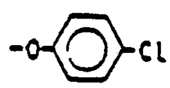 | 72 |
| 28 | 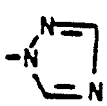 | 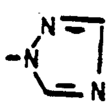 | 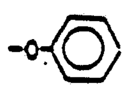 | 84 |
| 29 | 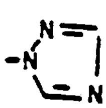 | 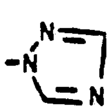 | 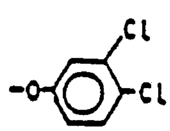 | 158 |

Tabelle 1 - Fortsetzung

| Beisp.-Nr. | $R^1$ | $R^2$ | $R^3$ | Schmp. (°C) oder $n_D^{20}$ |
|---|---|---|---|---|
| 30 | 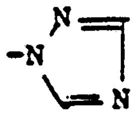 | 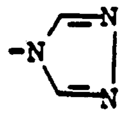 | 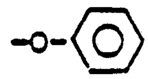 | 112 |
| 31 | " | 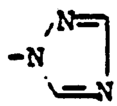 | 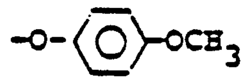 | 118 |
| 32 | " | 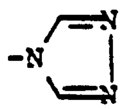 | 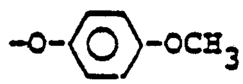 | 162 |
| 33 | " | " | 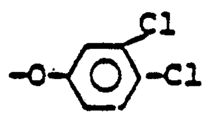 | 188 |
| 34 | " | 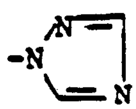 | 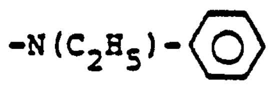 | 110 |
| 35 | " | " | 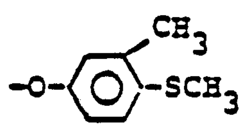 | 70 |
| 36 | " | 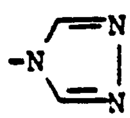 | 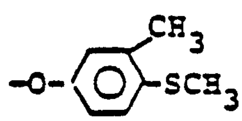 | 138 |
| 37 | " | 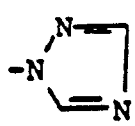 | 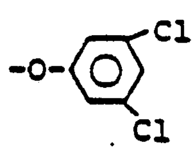 | 162 |
| 38 | " | 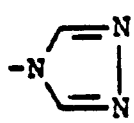 | 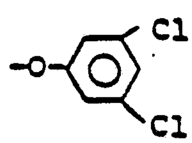 | 174 |

Tabelle 1 – Fortsetzung

| Beisp. Nr. | $R^1$ | $R^2$ | $R^3$ | Schmp. (°C) oder $n_D^{20}$ |
|---|---|---|---|---|
| 39 | 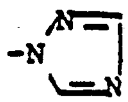 | 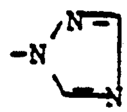 | 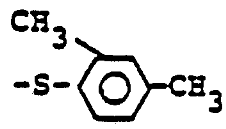 | 134 |
| 40 | " | 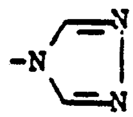 | 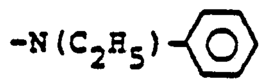 | 84 |
| 41 | " | 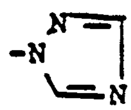 | 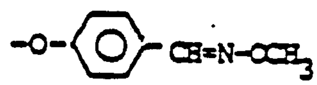 | 120 |
| 42 | " | " | 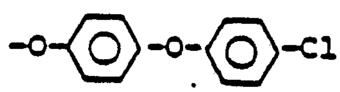 | 74 |
| 43 | " | 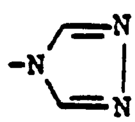 | 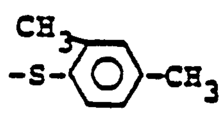 | 95 |
| 44 | " | 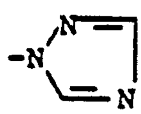 | 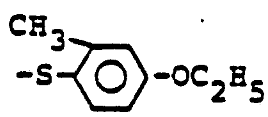 | 75 |
| 45 | " | 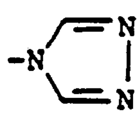 | 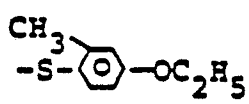 | 93 |
| 46 | " | 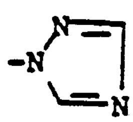 | $-S-C_2H_5$ | 46 |

Tabelle 1 - Fortsezung

| Bsisp.-Nr. | $R^1$ | $R^2$ | $R^3$ | Smp. (°C) oder $n_D^{20}$ |
|---|---|---|---|---|
| 47 | 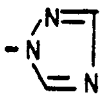 | 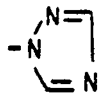 | 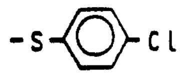 | 75 |
| 48 | '' | '' | 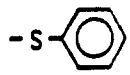 | 70 |
| 49 | '' | 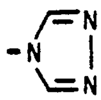 | 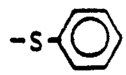 | 90 |
| 50 | '' | 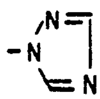 | 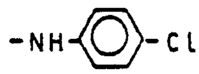 | 116 |
| 51 | '' | 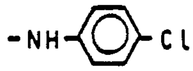 | 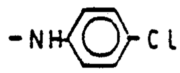 | 128 |
| 52 | '' | 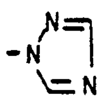 | 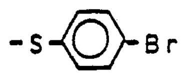 | 142 |
| 53 | '' | 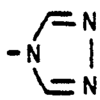 | 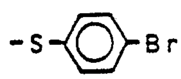 | 186 |
| 54 | '' | 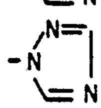 | 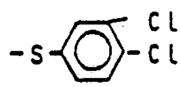 | 149 |
| 55 | '' | 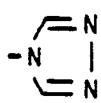 | 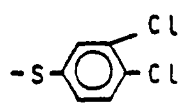 | 155 |
| 56 | '' | 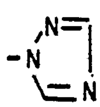 | 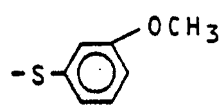 | 1,5820 |
| 57 | '' | 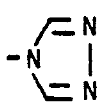 | 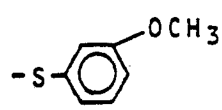 | 45 |
| 58 | '' | 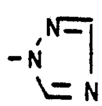 | 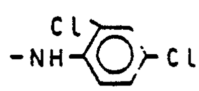 | 130 |

Tabelle 1 - Fortsetzung

| Bsp. Nr. | $R^1$ | $R^2$ | 3 | Schmp.(°C) oder $n_D^{20}$ |
|---|---|---|---|---|
| 59 | 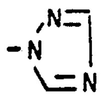 | 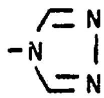 | 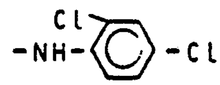 | 150 |
| 60 | " | 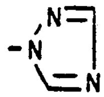 | 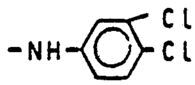 | 126 |
| 61 | " | 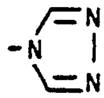 | 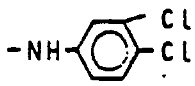 | 95 |
| 62 | " | 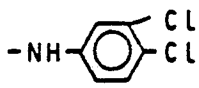 | 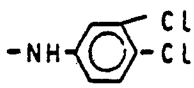 | 170 |
| 63 | " | 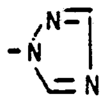 | 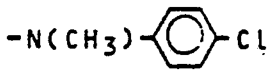 | 142 |
| 64 | " | 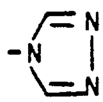 | 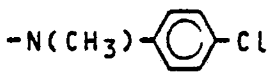 | 177 |
| 65 | " | 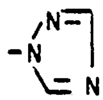 | 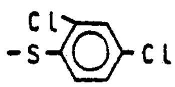 | 90 |
| 66 | " | 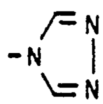 | 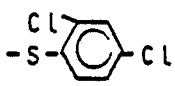 | 70 |
| 67 | " | 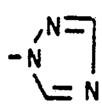 | 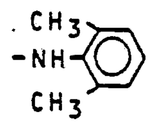 | 1,5620 |
| 68 | " | 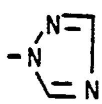 | 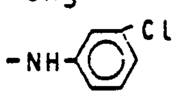 | 1,5698 |
| 69 | " | " | 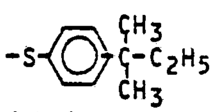 | 1,5600 |
| 70 | " | " | 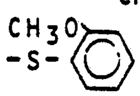 | 80 |
| 71 | " | 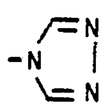 | 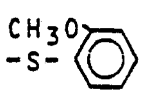 | 108 |
| 72 | " | 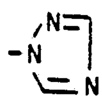 | 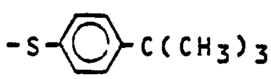 | 1,5608 |
| 73 | " | " | 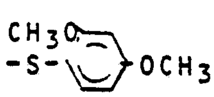 | 126 |

Tabelle 1 - Fortsetzung ---

| Bsp. Nr. | $R^1$ | $R^2$ | $R^3$ | Smp.(°C) oder $n_D^{20}$ |
|---|---|---|---|---|
| 74 | 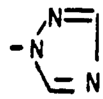 | 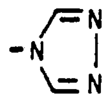 | 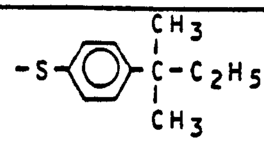 | 142 |
| 75 | '' | '' | 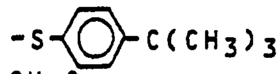 | 144 |
| 76 | '' | '' | 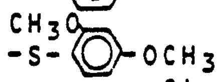 | zähes Oel |
| 77 | '' | 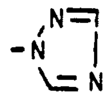 | 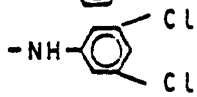 | 168 |
| 78 | '' | 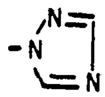 | 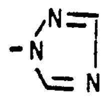 | 146 |
| 79 | '' | 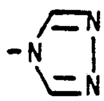 | 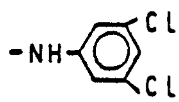 | 108 |
| 80 | '' | 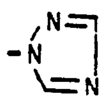 | 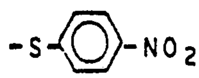 | 150 |
| 81 | '' | 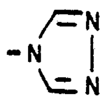 | 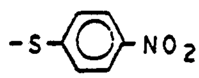 | 188 |
| 82 | '' | 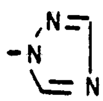 | 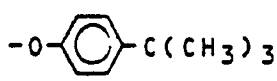 | 98 |
| 83 | '' | 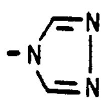 | 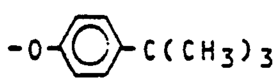 | 164 |
| 84 | '' | 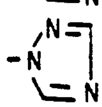 | 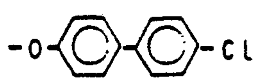 | 166 |
| 85 | '' | 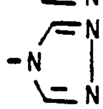 | 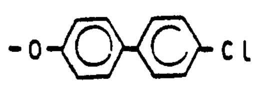 | 194 |
| 86 | '' | 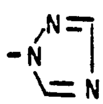 | 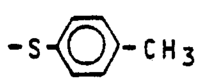 | 128 |
| 87 | '' | 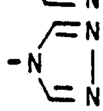 | 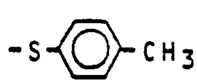 | 141 |
| 88 | '' | 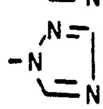 | 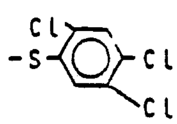 | 115 |

Tabelle 1 - Fortsetzung

| Bsp. Nr. | $R^1$ | $R^2$ | $R^3$ | Smp. (°C) bzw. $N_D^{20}$ |
|---|---|---|---|---|
| 89 | 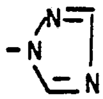 | 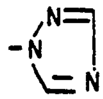 | 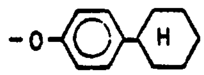 | 96 |
| 90 | '' | 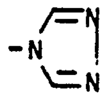 | 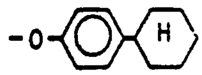 | 159 |
| 91 | '' | 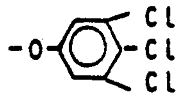 | 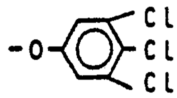 | 160 |
| 92 | '' | 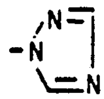 | 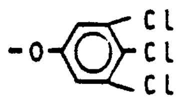 | 184 |
| 93 | '' | 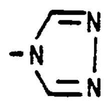 | 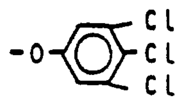 | 186 |
| 94 | '' | 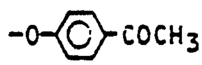 | 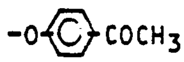 | 1,5750 |
| 95 | '' | 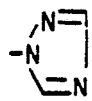 | 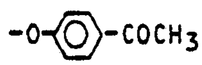 | 142 |
| 96 | '' | 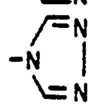 | 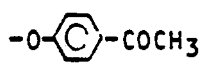 | 190 |
| 97 | '' | 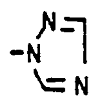 | 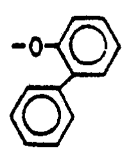 | 120 |
| 98 | '' | 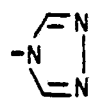 | 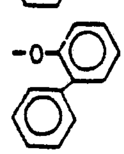 | 130 |
| 99 | '' | 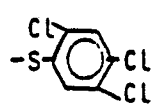 | 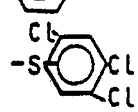 | 154 |
| 100 | '' | 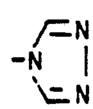 | 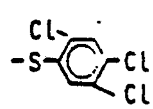 | 80 |

Tabelle 1 - Fortsetzung

| Bsp. Nr. | $R^1$ | $R^2$ | $R^3$ | Smp.(°C) bzw. $n_D^{20}$ |
|---|---|---|---|---|
| 101 | 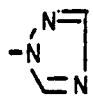 | 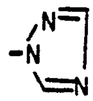 | 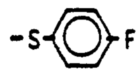 | 114 |
| 102 | " | 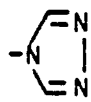 | 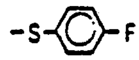 | 169 |
| 103 | " | 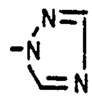 | 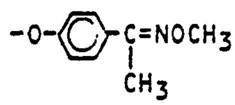 | 140 |
| 104 | " | 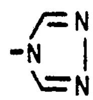 | 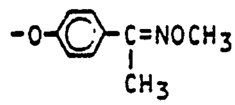 | 154 |
| 105 | " | 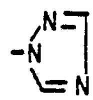 | 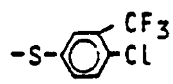 | 50 |
| 106 | " | 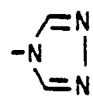 | 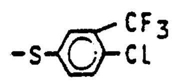 | 75 |
| 107 | " | 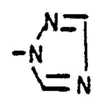 | $-S-(CH_2)_{11}-CH_3$ | 70 |
| 108 | 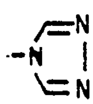 | 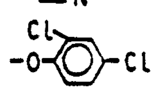 | 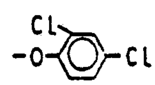 | 169 |
| 109 | " | $-S-(CH_2)_{11}-CH_3$ | $-S-(CH_2)_{11}-CH_3$ | 84 |
| 110 | " | 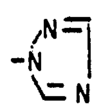 | $-S-(CH_2)_{11}-CH_3$ | 82 |
| 111 | 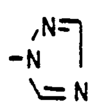 | $-S-(CH_2)_{11}-CH_3$ | $-S-(CH_2)_{11}-CH_3$ | 51 |
| 112 | " | 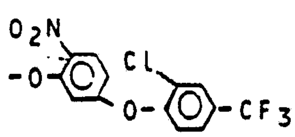 | 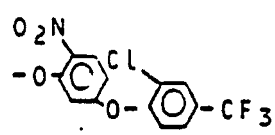 | 96 |
| 113 | " | 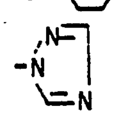 | 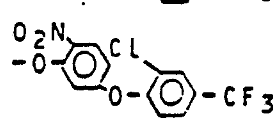 | 92 |
| 114 | " | 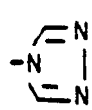 | 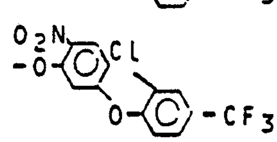 | 130 |

Tabelle 1 - Fortsetzung

| Bsp. Nr. | $R^1$ | $R^2$ | $R^3$ | Smp.(°C) bzw. $n_D^{20}$ |
|---|---|---|---|---|
| 115 | 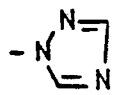 | 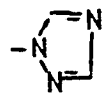 | $-S-C(CH_3)_3$ | 70 |
| 116 | " | 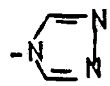 | $-S-C(CH_3)_3$ | 91 |
| 117 | " | 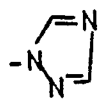 | 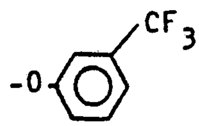 | 97 |
| 118 | " | 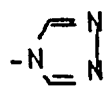 | 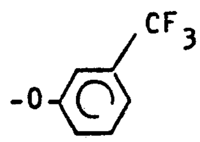 | 110 |
| 119 | " | 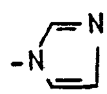 | 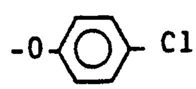 | 100 |
| 120 | 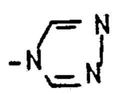 | 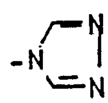 | 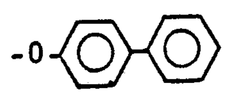 | 275 |
| 121 | 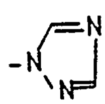 | 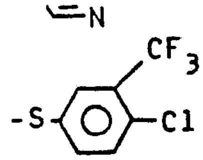 | 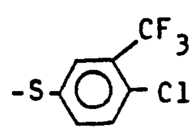 | 1.5674 |

Entsprechend den Herstellungsbeispielen und den allgemeinen Verfahrensangaben werden die folgenden Zwischenprodukte der allgemeinen Formel (IV)

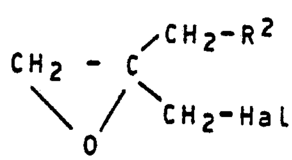 (IV)

in Tabelle 2 erhalten:

Tabelle 2

| Bsp. Nr. | $R^2$ | Hal | Siedepunkt (°C) /mbar bzw. $n_D^{20}$ |
|---|---|---|---|
| (IV-5) | 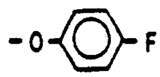 | Cl | 100/0,2 |
| (IV-6) | 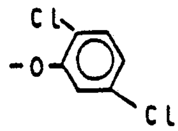 | Cl | 145/0,1 |
| (IV-7) | 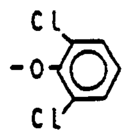 | Cl | 155/0,3 |
| (IV-8) | 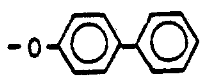 | Cl | 1,5842 |
| (IV-9) | 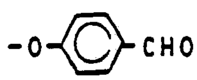 | Cl | 190/0,2 |
| (IV-10) | 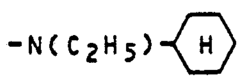 | Cl | 100/0,1 |
| (IV-11) | 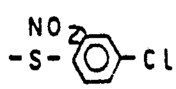 | Cl | 1,6270 |
| (IV-12) | 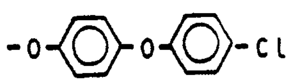 | Cl | 1,5442 |
| (IV-13) | 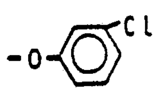 | Cl | 140/0,5 |
| (IV-14) | 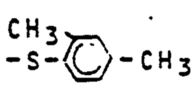 | Cl | 122/0,1 |

Tabelle 2 - Fortsetzung

| Bsp. Nr. | $R^2$ | Hal | Siedepunkt (°C)/mbar bzw. $n_D^{20}$ |
|---|---|---|---|
| (IV-15) | 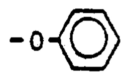 | Cl | 110/0,1 |
| (IV-16) | 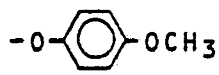 | Cl | 120/0,2 |
| (IV-17) | 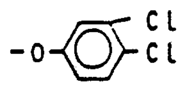 | Cl | 140/0,2 |
| (IV-18) | 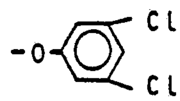 | Cl | 140/0,2 |
| (IV-19) | 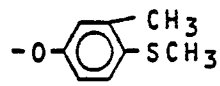 | Cl | 160/0,2 |
| (IV-20) | 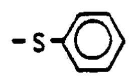 | Cl | 120/0,3 |
| (IV-21) | 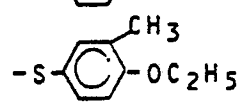 | Cl | 138/0,1 |
| (IV-22) | $-S-C_2H_5$ | Cl | 1,4968 |
| (IV-23) | 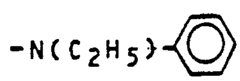 | Cl | 130/0,3 |
| (IV-24) | 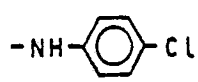 | Cl | 1,5843 |
| (IV-25) | 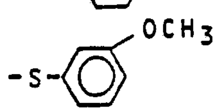 | Cl | 160/0,3 |
| (IV-26) | 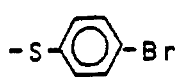 | Cl | 158/0,3 |
| (IV-27) | 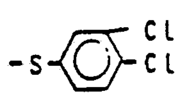 | Cl | 155/0,3 |
| (IV-28) | 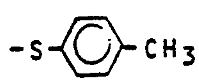 | Cl | 1,5575 |
| (IV-29) | $-S-(CH_2)_{11}-CH_3$ | Cl | 1,4775 |

Tabelle 2 - Fortsetzung

| Bsp. Nr. | $R^2$ | Hal | Siedepunkt (°C) /mbar bzw. $n_D^{20}$ |
|---|---|---|---|
| (IV-30) | 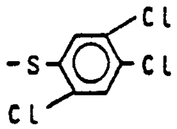 | Cl | 1,6065 |
| (IV-31) | 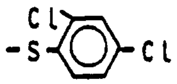 | Cl | 1,5951 |
| (IV-32) | 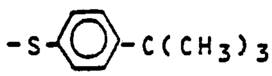 | Cl | 1,5514 |
| (IV-33) | 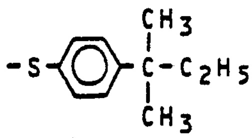 | Cl | 1,5408 |
| (IV-34) | 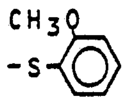 | Cl | 1,5748 |
| (IV-35) | 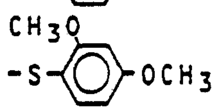 | Cl | 1,5613 |
| (IV-36) | 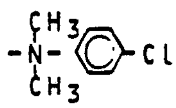 | Cl | 1,5738 |
| (IV-37) | 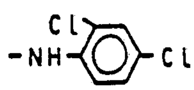 | Cl | 1,5805 |
| (IV-38) | 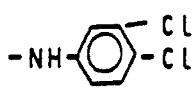 | Cl | 1,5858 |
| (IV-39) | 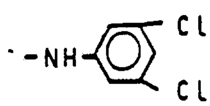 | Cl | 1,5940 |
| (IV-40) | 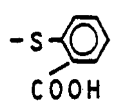 | Cl | 1,5840 |
| (IV-41) | 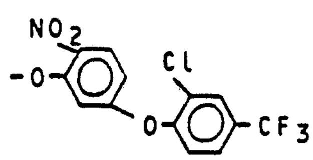 | Cl | 1,5531 |
| (IV-42) | $-SC(CH_3)_3$ | Cl | 1,4871 |
| (IV-43) | 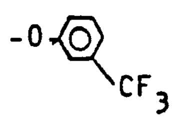 | Cl | 1,4880 |

Entsprechend den Herstellungsbeispielen und den allgemeinen Verfahrensangaben werden die folgenden Zwischenprodukte der allgemeinen Formel (V)

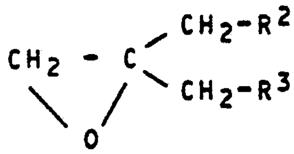 (V)

in Tabelle 3 erhalten:

Tabelle 3

| Bsp. | $R^2$ | $R^3$ | Schmelzpunkt (°C) bzw. $n_D^{20}$ |
|---|---|---|---|
| (V-2) | 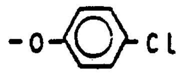 | 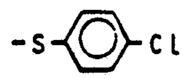 | 1,6347 |
| (V-3) | 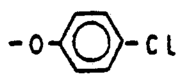 | 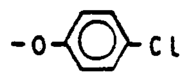 | 84 |
| (V-4) | 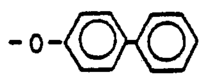 | 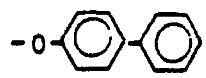 | 176 |
| (V-5) | 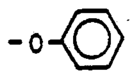 | 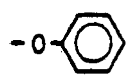 | 1,5612 |
| (V-6) | 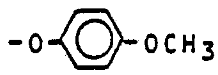 | 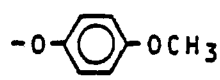 | 100 |
| (V-7) | 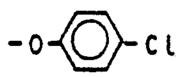 | 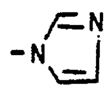 | 1,5590 |
| (V-8) | 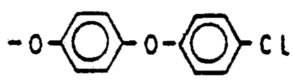 | 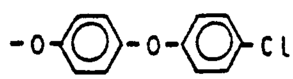 | 50 |
| (V-9) | 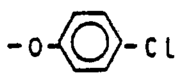 | 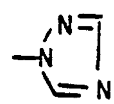 | 96 |

Die Herstellung der gegebenenfalls als vorprodukte zu verwendenden Propen-Derivate der Formeln (VIII) und (IX) sei anhand der folgenden Beispiele erläutert:

**Beispiel** α

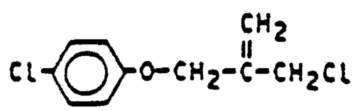

(Beispiel VIII-1)

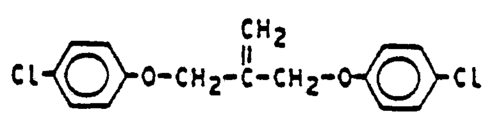

(Beispiel IX-1)

Eine Lösung von 64,25 g (0,5 Mol) 4-Chlorphenol in 100 ml Aceton wird bei 60°C zu einem Gemisch von 125 g (1 Mol) 3-Chlor-2-chlormethyl-propen und 34,5 g (0,25 Mol) Kaliumcarbonat in 500 ml Aceton getropft. Man läßt 18 Stunden bei dieser Temperatur nachrühren, filtriert und engt das Filtrat ein. Der ölige Rückstand wird in Methylenchlorid gelöst, einmal mit 200 ml 10 %-iger Natronlauge und danach zweimal mit je 500 ml Wasser gewaschen, über Natriumsulfat getrocknet und eingeengt. Der ölige Rückstand wird destilliert. Man erhält 43,2 g (41,5 % der Theorie) 2-Chlormethyl-3-(4-chlorphenoxy)-propen (Beispiel VIII-1) vom Siedepunkt 80 bis 90°C/0,05 mbar.

Aus dem Destillationsrückstand erhält man nach Verrühren mit Diisopropylether 10,7 g 3-(4-Chlorphenoxy)-2-(4-chlorphenoxymethyl)-propen (Beispiel IX-1) vom Schmelzpunkt 72°C.

**Bespiel β**

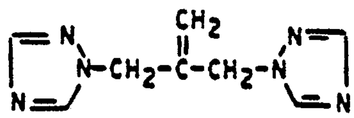

(Beispiel VIII-2)

Ein Gemisch von 25 g (0,2 Mol) 3-Chlor-2-chlormethyl-propen, 27,6 g (0,4 Mol) 1,2,4-Triazol und 27,6 g (0,2 Mol) Kaliumcarbonat in 200 ml Aceton wird 15 Stunden unter Rückfluß erhitzt. Nach dem Abkühlen wird filtriert und das Filtrat im Vakuum eingeengt. Der ölige Rückstand wird chromatographiert (Kieselgel 60, Merck; Chloroform).

Man erhält 23 g (63 % der Theorie) 3-(1,2,4-Triazol-1-yl)-2-(1,2,4-triazol-1-yl-methyl)-propen vom Schmelzpunkt 52° C.

**Verwendungsbeispiele**

In den nachfolgenden Beispielen werden die nachstehend angegebenen Verbindungen als Vergleichssubstanzen eingesetzt:

(A) 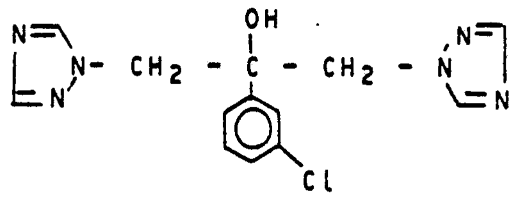

(B) 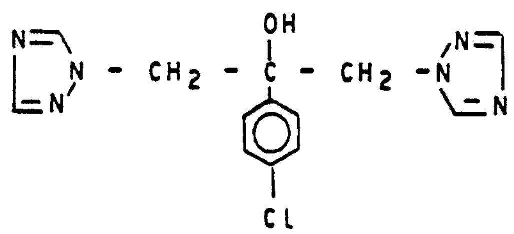

(C) 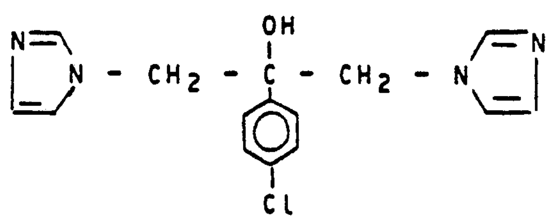

**Beispiel A**

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge pflanzen mit der Wirkstoffzubereitung

taufeucht. Nach Antrocknen des Spritzbelages werden die pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach ler Inokulation erfolgt die Auswertung.

Eine deutliche Überlegenheit in der Wirksamkeit gegenüber dem Stand der Technik zeigen in diesem Test z. B. die Verbindungen gemäß folgender Herstellungsbeispiele: 5, 3, 11, 14, 23, 31, 8, 29, 60, 85, 88 und 92.

T a b e l l e: A

Erysiphe-Test (Gerste) / protektiv

| W i r k s t o f f | Wirkstoff-konzentration in der Spritzbrühe in Gew.-% | Krankheitsbefall in % der unbehandelten Kontrolle |
|---|---|---|
| 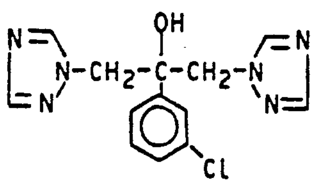 (A) (bekannt) | 0,0025 | 100 |
| 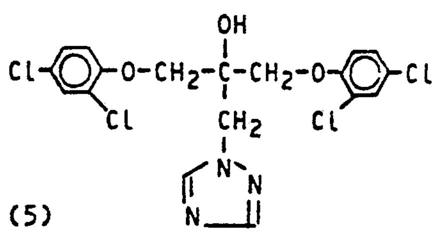 (5) | 0,0025 | 33,7 |
| 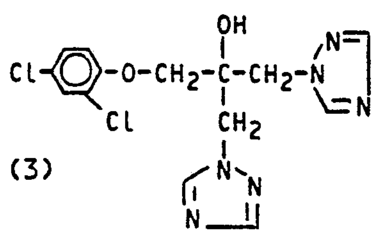 (3) | 0,0025 | 0,0 |
| 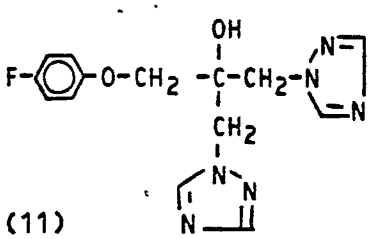 (11) | 0,0025 | 25,0 |

T a b e l l e: A (Fortsetzung)

Erysiphe-Test (Gerste) / protektiv

| W i r k s t o f f | Wirkstoffkonzentration in der Spritzbrühe in Gew.-% | Krankheitsbefall in % der unbehandelten Kontrolle |
|---|---|---|
| 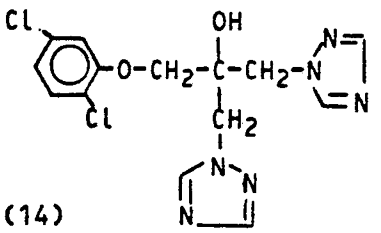 (14) | 0,0025 | 25,0 |
| 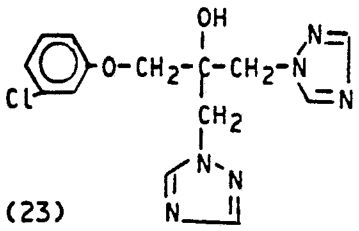 (23) | 0,0025 | 12,5 |
| 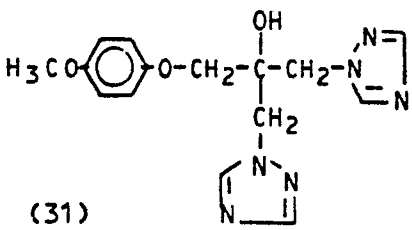 (31) | 0,0025 | 16,2 |

Tabelle: A (Fortsetzung)

Erysiphe-Test (Gerste) / protektiv

| Wirkstoff | Wirkstoffkonzentration in der Spritzbrühe in Gew.-% | Krankheitsbefall in % der unbehandelten Kontrolle |
|---|---|---|
| 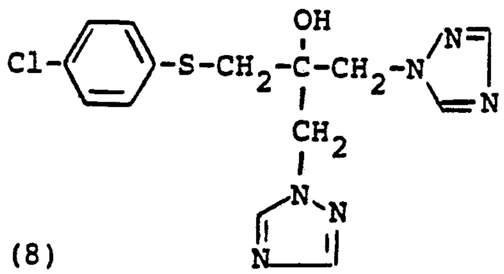 (8) | 0,0025 | 25,0 |
| 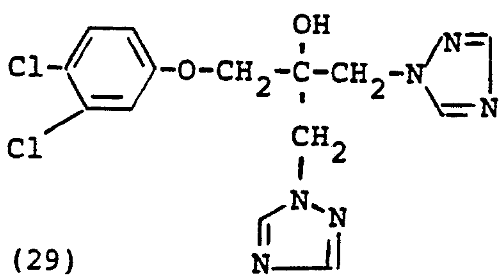 (29) | 0,0025 | 25,0 |
| 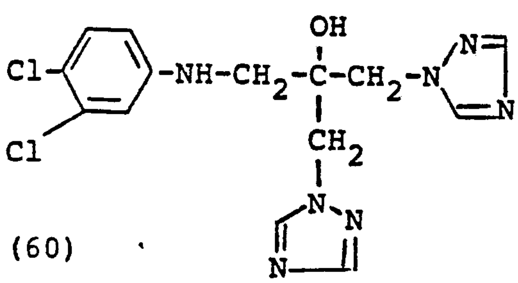 (60) | 0,0025 | 16,2 |

Tabelle: A (Fortsetzung)

Erysiphe-Test (Gerste) / protektiv

| Wirkstoff | Wirkstoffkonzentration in der Spritzbrühe in Gew.-% | Krankheitsbefall in % der unbehandelten Kontrolle |
|---|---|---|
| 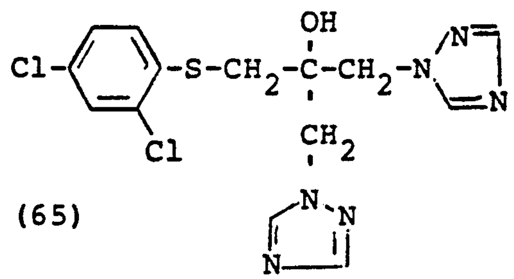 (65) | 0,0025 | 3,7 |
| 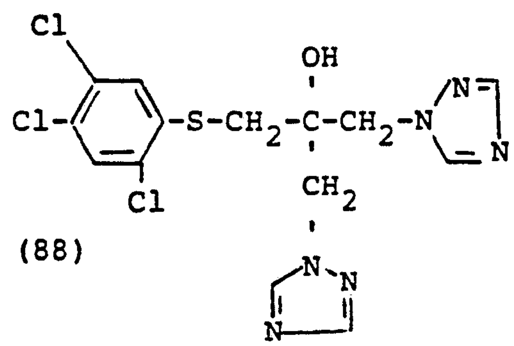 (88) | 0,0025 | 12,5 |
| 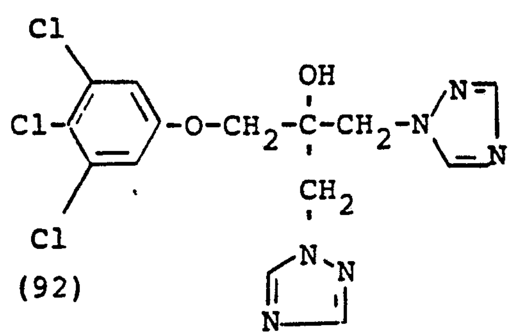 (92) | 0,0025 | 25,0 |

**Beispiel B**

Leptosphaeria nodorum - Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20°C und 100 % relativer Luftfeuchtigkeit in einer Inkubationskabine.

Die Pflanzen werden im Gewächshaus bei einer Temperatur von ca. 15°C und einer relativen Luftfeuchtigkeit von ca. 80 % aufgestellt.

10 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgendem Herstellungsbeispiel: 5.

Tabelle: B

Leptosphaeria nodorum - Test (Weizen) / protektiv

| Wirkstoff | Wirkstoffkonzentration in der Spritzbrühe in Gew. % | Krankheitsbefall in % der unbehandelten Kontrolle |
|---|---|---|
| 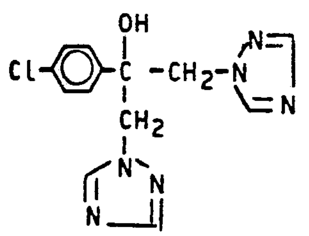 (B) (bekannt) | 0,025 | 100 |
| 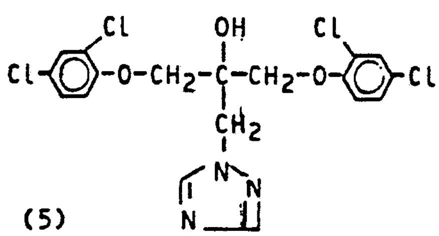 (5) | 0,025 | 33,3 |

**Beispiel C**

Sphaerotheca-Test (Gurke) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdunnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca

fuliginea bestäubt.

Die Pflanzen werden anschließend bei 23 bis 24° C und bei einer relativen Luftfeuchtigkeit von ca. 75 % im Gewächshaus aufgestellt.

10 Tage nach der Inokulation erfolgt die Auswertung.

Eine deutliche Überlegenheit in der Wirksamkeit gegenüber dem Stand der Technik zeigen in diesem Test z. B. die Verbindungen gemäß folgender Herstellungsbeispiele: 6, 3, 8, 12 und 29.

## Tabelle C

Sphaerotheca-Test (Gurke) / protektiv

| Wirkstoff | Befall in % bei einer Wirkstoffkonzentration von 5 ppm |
|---|---|
| 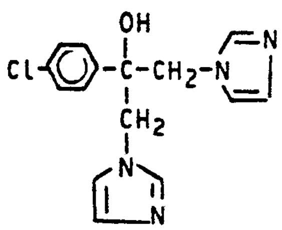 (C) (bekannt) | 100 |
| 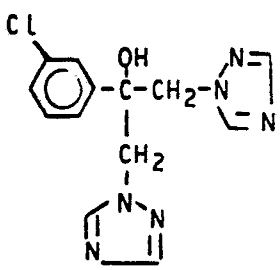 (A) (bekannt) | 43 |
| 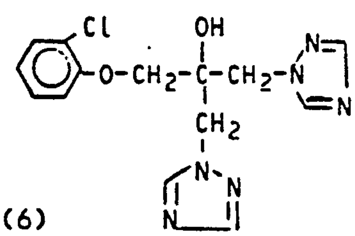 (6) | 10 |
| 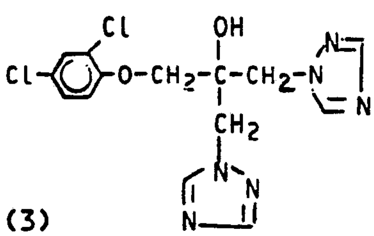 (3) | 10 |

<u>Tabelle</u> C (Fortsetzung)

Sphaerotheca-Test (Gurke) / protektiv

| Wirkstoff | Befall in % bei einer Wirkstoffkonzentration von 5 ppm |
|---|---|
| 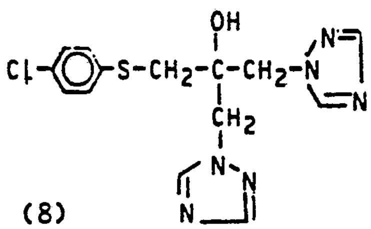 (8) | 20 |
| 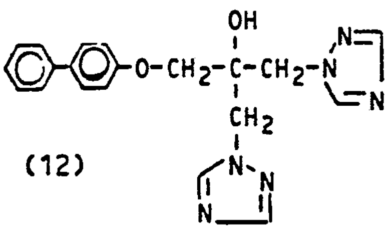 (12) | 30 |
| 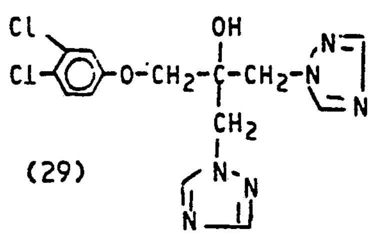 (29) | 13 |

**Beispiel D**

<u>Cochliobolus sativus-Test (Gerste) / protektiv</u>

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von

Cochliobolus sativus besprüht. Die Pflanzen verbleiben 48 Stunden bei 20°C und 100 % rel. Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber den Stand der Technik zeigen bei diesem Test z. B. die Verbindungen gemäß folgender Herstellungsbeispiele: 12, 52, 54, 69, 72, 82, 86, 89, 91, 94, 95, 96, 97, 98, 99, 100, 101, 103.

## Tabelle D

Cochliobolus sativus-Test (Gerste) /protektiv

| Wirkstoff | Wirkstoffkonzentration in der Spritzbrühe in Gew.-% | Krankheitsbefall in % der unbehandelten Kontrolle |
|---|---|---|
| 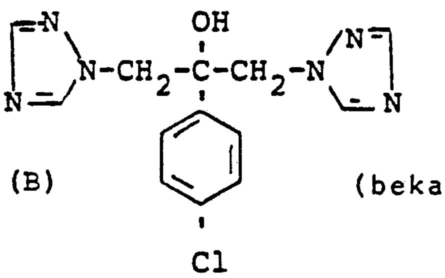 (B) (bekannt) | 0,025 | 100 |
| 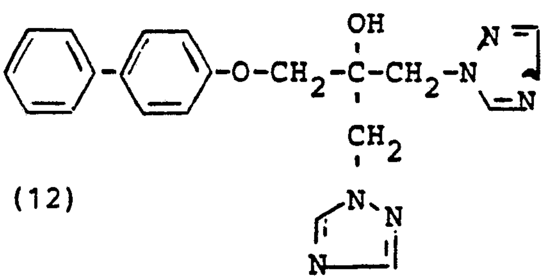 (12) | 0,025 | 16,2 |
| 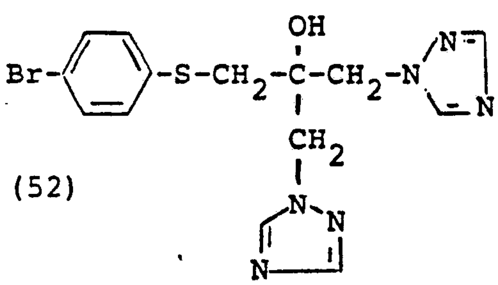 (52) | 0,025 | 0,0 |
| 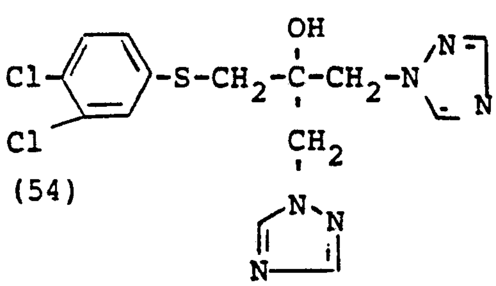 (54) | 0,025 | 0,0 |

Tabelle D (Forts.)

Cochliobolus sativus-Test (Gerste) /protektiv

| Wirkstoff | Wirkstoffkonzentration in der Spritzbrühe in Gew.-% | Krankheitsbefall in % der unbehandelten Kontrolle |
|---|---|---|
| 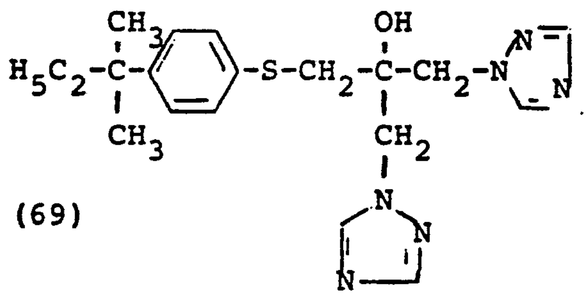 (69) | 0,025 | 12,5 |
| 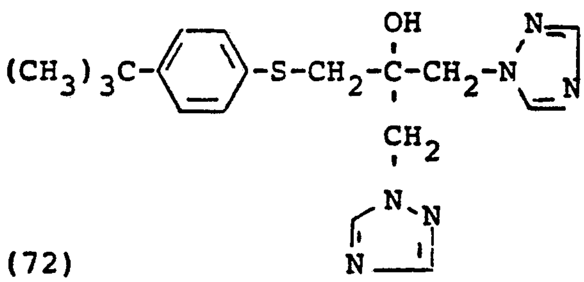 (72) | 0,025 | 12,5 |
| 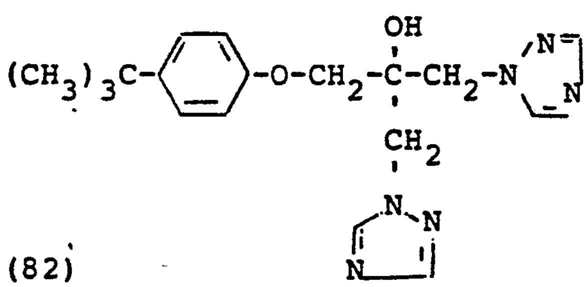 (82) | 0,025 | 50,0 |

Tabelle D (Forts).

Cochliobolus sativus-Test (Gerste) /protektiv

| Wirkstoff | Wirkstoffkonzentration in der Spritzbrühe in Gew.-% | Krankheitsbefall in % der unbehandelten Kontrolle |
|---|---|---|
| 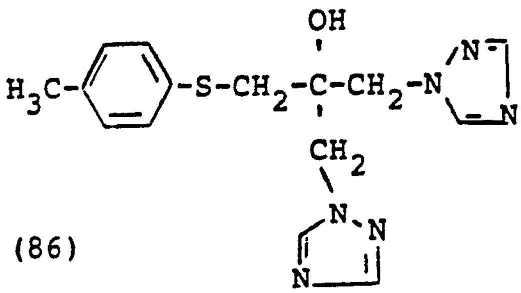 (86) | 0,025 | 12,5 |
| 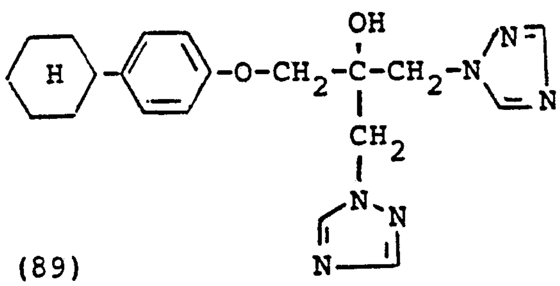 (89) | 0,025 | 25,0 |
| 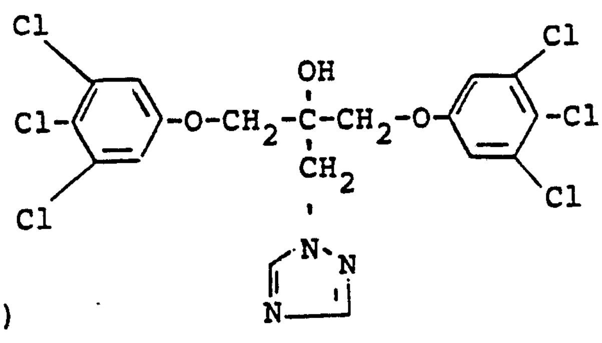 (91) | 0,025 | 50,0 |

Tabelle D (Forts.)

Cochliobolus sativus-Test (Gerste) /protektiv

| Wirkstoff | | Wirkstoffkonzentration in der Spritzbrühe in Gew.-% | Krankheitsbefall in % der unbehandelten Kontrolle |
|---|---|---|---|
| 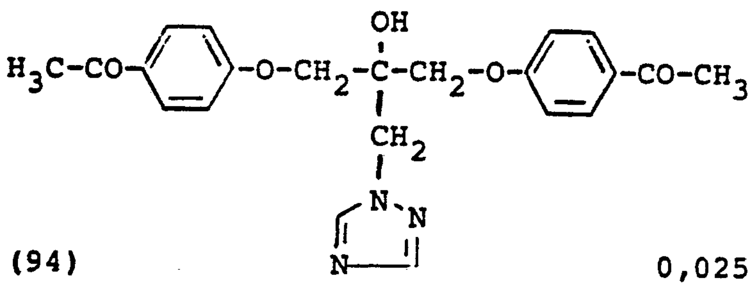 | (94) | 0,025 | 50,0 |
| 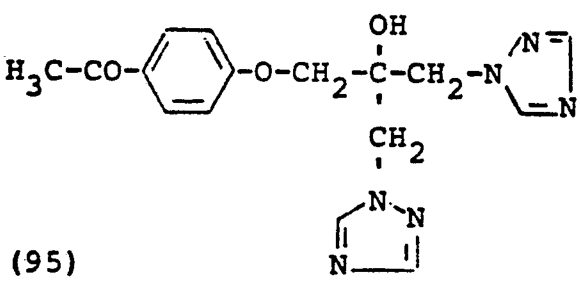 | (95) | 0,025 | 50,0 |
| 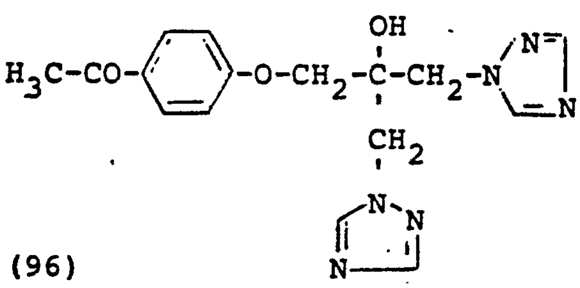 | (96) | 0,025 | 50,0 |

Tabelle D (Forts.)

Cochliobolus sativus-Test (Gerste) /protektiv

| Wirkstoff | | Wirkstoffkonzentration in der Spritzbrühe in Gew.-% | Krankheitsbefall in % der unbehandelten Kontrolle |
|---|---|---|---|
| (97) | 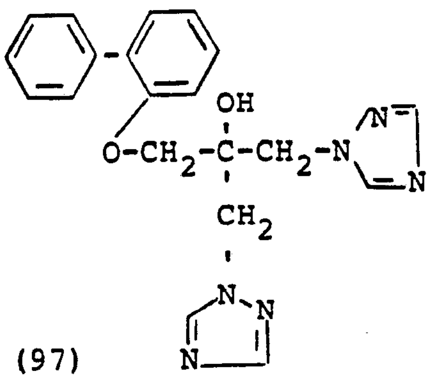 | 0,025 | 50,0 |
| (98) | 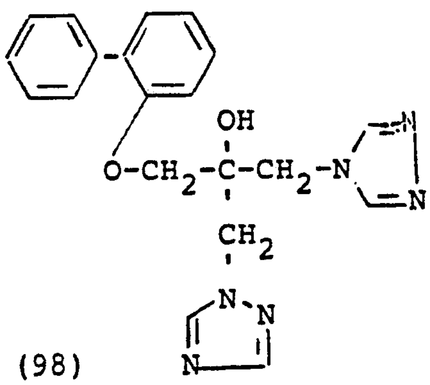 | 0,025 | 25,0 |
| (99) | 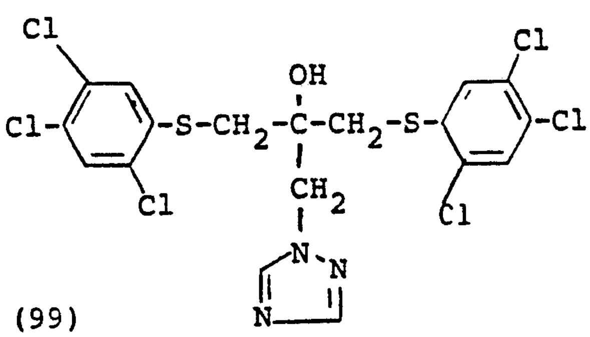 | 0,025 | 25,0 |

T a b e l l e  D (Forts.)

Cochliobolus sativus-Test (Gerste) /protektiv

| Wirkstoff | Wirkstoff-konzentration in der Spritzbrühe in Gew.-% | Krankheits-befall in % der unbehandelten Kontrolle |
|---|---|---|
| 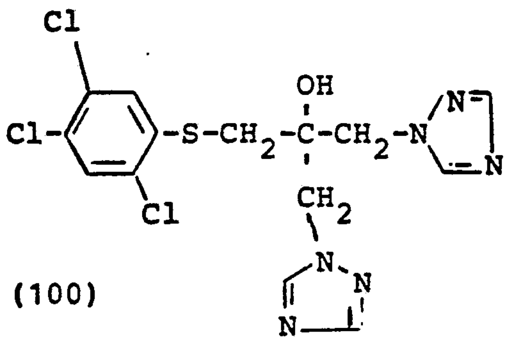 (100) | 0,025 | 33,7 |
| 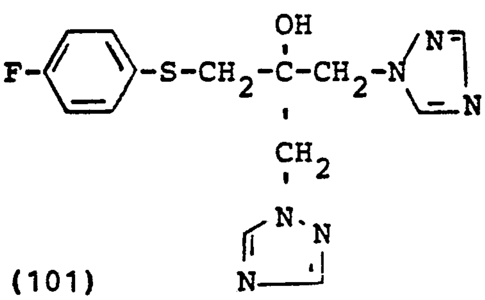 (101) | 0,025 | 50,0 |
| 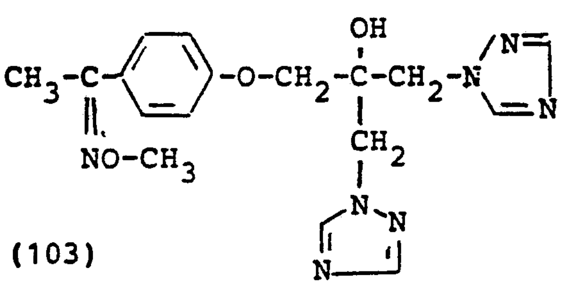 (103) | 0,025 | 50,0 |

## Patentansprüche

1. Verwendung von substituierten tert.-Butanol-Derivaten der Formel

$$\begin{array}{c} OH \\ | \\ R^1 - CH_2 - C - CH_2 - R^2 \\ | \\ CH_2 \\ | \\ R^3 \end{array} \qquad (I)$$

in welcher

$R^1$, $R^2$ und $R^3$ gleich oder verschieden sind und für Azolyl, gegebenenfalls substituiertes Phenoxy, gegebenenfalls substituiertes Phenylthio, Alkylthio, Alkenyl, Alkinyl, gegebenenfalls substituiertes Phenylacetylenyl, Alkylamino, Dialkylamino, gegebenenfalls substituiertes Phenylamino, gegebenenfalls substituiertes Phenyl-N-alkyl-amino, gegebenenfalls substituiertes Cycloalkylamino, gegebenenfalls substituiertes Cycloalkyl-N-alkyl-amino, Aminoethoxy, Alkylaminoethoxy oder Dialkylaminoethoxy stehen,

sowie von deren Säureadditions-Salzen und Metallsalz-Komplexen als Pflanzenschutzmittel.

2. Verwendung gemäß Anspruch 1, dadurch gekennzeichnet, daß man substituierte tert.-Butanol-Derivate der Formel (I) einsetzt, in welcher

$R^1$ und $R^2$ gleich oder verschieden sind und für Imidazol-1-yl, 1,2,4-Triazol-1-yl; 1,2,4-Triazol-4-yl oder Pyrazol-1-yl stehen und

$R^3$ für jeweils gegebenenfalls einfach oder mehrfach, gleich oder verschieden substituiertes Phenoxy, Phenylthio, Phenylamino oder Phenyl-N-alkyl-amino mit 1 bis 4 Kohlenstoffatomen im Alkylteil steht, wobei die genannten Gruppen im Phenylteil substituiert sein können durch Halogen,.Alkyl mit 1 bis 8 Kohlenstoffatomen, Alkoxy und Alkylthio mit jeweils 1 bis 4 Kohlenstoffatomen; durch Halogenalkyl, Halogenalkoxy und Halogenalkylthio mit jeweils 1 bis 4 Kohlenstoffatomen und 1 bis 5 gleichen oder verschiedenen Halogenatomen, wie Fluor- und Chloratomen, Cycloalkyl mit 5 bis 6 Kohlenstoffatomen; ferner durch Nitro, Cyano, Hydroxycarbonyl, Alkylcarbonyl oder Alkoxycarbonyl mit jeweils 1 bis 4 Kohlenstoffatomen im Alkylteil, gegebenenfalls einfach oder mehrfach, gleich oder verschieden durch Halogen, Nitro, Trifluormethyl und Alkyl mit 1 bis 2 Kohlenstoffatomen substituiertes Phenyl oder Phenoxy, durch die Aldehydgruppe sowie den Oxim- bzw. Oximether-Rest.

3. Verwendung gemäß Anspruch 1, dadurch gekennzeichnet, daß man substituierte tert.-Butanol-Derivate der Formel (I) einsetzt, in welcher

$R^1$ und $R^2$ gleich oder verschieden sind und für Imidazol-1-yl; 1,2,4-Triazol-1-yl; 1,2,4-Triazol-4-yl oder Pyrazol-1-yl stehen und

$R^3$ für jeweils gegebenenfalls einfach bis dreifach, gleich oder verschieden substituiertes Phenoxy, Phenylthio, Phenylamino oder Phenyl-N-alkyl-amino mit 1 bis 2 Kohlenstoffatomen im Alkylteil steht, wobei die genannten Gruppen im Phenylteil substituiert sein können durch Fluor, Chlor, Brom, Methyl, Ethyl, Isopropyl, tert.-Butyl, 2-Methyl-but-2-yl, Methoxy, Ethoxy, Methylthio, Trifluormethyl, Trifluormethoxy, Trifluormethylthio, Cyclohexyl, Nitro, Cyano, Hydroxycarbonyl, Methylcarbonyl, Methoxycarbonyl, Ethoxycarbonyl, durch jeweils gegebenenfalls einfach bis dreifach, gleich oder verschieden durch Chlor, Nitro, Trifluormethyl oder Methyl substituiertes Phenyl oder Phenoxy, die Aldehydgruppe, die Hydroxyiminomethyl- und Methoximinomethyl-Gruppe.

4. Verwendung von substituierten tert.-Butanol-Derivaten der Formel (I) gemäß Anspruch 1 zur Bekämpfung von Pilzen im Pflanzenschutz.

**Claims**

1. Use of substituted tert.-butanol derivatives of the formula

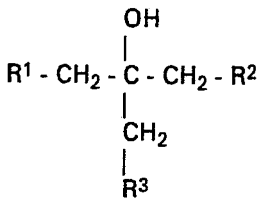

(I)

in which

R¹, R² and R³ are identical or different and represent azolyl, optionally substituted phenoxy, optionally substituted phenylthio, alkylthio, alkenyl, alkinyl, optionally substituted phenylacetylenyl, alkylamino, dialkylamino, optionally substituted phenylamino, optionally substituted phenyl-N-alkyl-amino, optionally substituted cycloalkylamino, optionally substituted cycloalkyl-N-alkyl-amino, aminoethoxy, alkylaminoethoxy or dialkylaminoethoxy,

and of acid addition salts and metal salt complexes thereof as plant protection agents.

2. Use according to Claim 1, characterized in that substituted tert.-butanol derivatives of the formula (I) are used,
in which

$R^1$ and $R^2$ are identical or different and represent imidazol-1-yl, 1,2,4-triazol-1-yl; 1,2,4-triazol-4-yl or pyrazol-1-yl and

$R^3$ represents phenoxy, phenylthio, phenylamino or phenyl-N-alkyl-amino with 1 to 4 carbon atoms in the alkyl part, each of which is optionally monosubstituted or polysubstituted by identical or different substituents, it being possible for the groups mentioned to be substituted in the phenyl part by halogen, alkyl with 1 to 6 carbon atoms, alkoxy and alkylthio with in each case 1 to 4 carbon atoms; by halogenoalkyl, halogenoalkoxy and halogenoalkylthio with in each case 1 to 4 carbon atoms and 1 to 5 identical or different halogen atoms, such as fluorine and chlorine atoms, cycloalkyl with 5 to 6 carbon atoms; furthermore by nitro, cyano, hydroxycarbonyl, alkylcarbonyl or alkoxycarbonyl with in each case 1 to 4 carbon atoms in the alkyl part, phenyl or phenoxy which is optionally monosubstituted or polysubstituted by identical or different substituents from the group comprising halogen, nitro, trifluoromethyl and alkyl with 1 or 2 carbon atoms, by the aldehyde group or the oxime or oxime ether radical.

3. Use according to Claim 1, characterized in that substituted tert.-butanol derivatives of the formula (I) are used,
in which

$R^1$ and $R^2$ are identical or different and represent imidazol-1-yl; 1,2,4-triazol-1-yl; 1,2,4-triazol-4-yl or pyrazol-1-yl and

$R^3$ represents phenoxy, phenylthio, phenylamino or phenyl-N-alkyl-amino with 1 or 2 carbon atoms in the alkyl part, each of which is optionally mono-, di- or trisubstituted by identical or different substituents, it being possible for the groups mentioned to be substituted in the phenyl part by fluorine, chlorine, bromine, methyl, ethyl, isopropyl, tert.-butyl, 2-methyl-but-2-yl, methoxy, ethoxy, methylthio, trifluoromethyl, trifluoromethoxy, trifluoromethylthio, cyclohexyl, nitro, cyano, hydroxycarbonyl, methylcarbonyl, methoxycarbonyl or ethoxycarbonyl, or phenyl or phenoxy, each of which is optionally mono-, di- or trisubstituted by identical or different substituents from the group comprising chlorine, nitro, trifluoromethyl and methyl, or the aldhyde group, the hydroxyiminomethyl group or methoximinomethyl group.

4. Use of substituted tert.-butanol derivatives of the formula (I) according to Claim 1 for combating fungi in plant protection.

## Revendications

1. Utilisation d'un dérivé substitué du tertiobutanol, de formule

$$R^1 - CH_2 - \underset{\substack{|\\ CH_2\\ |\\ R^3}}{\overset{\substack{OH\\ |}}{C}} - CH_2 - R^2 \qquad (I)$$

dans laquelle

$R^1$, $R^2$ et $R^3$ sont identiques ou différents et représentent chacun un groupe azolyle, un groupe phénoxy éventuellement substitué, un groupe phénylthio éventuellement substitué, un groupe alkylthio, alcényle, alcynyle, un groupe phénylacétylènyle éventuellement substitué, un groupe alkylamino, un groupe dialkylamino, un groupe phénylamino éventuellement substitué, un groupe phényl-N-alkyl-amino éventuellement substitué, un groupe cycloalkylamino éventuellement substitué, un groupe cycloalkyl-N-alkyl-amino éventuellement substitué, un groupe aminoéthoxy, alkylaminoéthoxy ou dialkylaminoéthoxy,

ainsi que de leurs sels d'addition d'acides et de leurs complexes avec des sels de métaux, comme produits phytosanitaires pour la protection des plantes.

2. Utilisation selon la revendication 1, caractérisée en ce que l'on utilise des dérivés substitués du tertiobutanol, de formule (I), dans laquelle:

$R^1$ et $R^2$ sont identiques ou différents et représentent chacun un groupe imidazol-1-yle, un groupe 1,2,4-triazol-1-yle; un groupe 1,2,4-triazol-4-yle ou pyrazol-1-yle, et

$R^3$ représente un groupe phénoxy, phénylthio, phénylamino ou phényl-N-alkyl-amino comportant 1 à 4 atomes de carbone dans la partie alkyle et dont chacun est éventuellement substitué, de façon identique ou différente, une ou plusieurs fois, et l'on peut citer, pour les groupes de substituants fixés sur la partie alkyle, un atome d'halogène, un groupe alkyle ayant 1 à 6 atomes de carbone, un groupe alcoxy et alkylthio ayant chacun 1 à 4 atomes de carbone, un groupe halogénoalkyle, halogénoalcoxy et halogénoalkylthio ayant chacun 1 à 4 atomes de carbone et comportant 1 à 5 atomes d'halogène identiques ou différents, comme des atomes de fluor et de chlore, un groupe cycloalkyle ayant 5 ou 6 atomes de carbone; ainsi qu'un groupe nitro, cyano, hydroxycarbonyle, alkylcarbonyle ou alcoxycarbonyle ayant chacun 1 à 4 atomes de carbone dans la partie alkyle, un groupe phényle ou phénoxy éventuellement substitué une ou plusieurs fois, de façon identique ou différente, par de l'halogène, par un groupe nitro, trifluorométhyle et alkyle ayant 1 ou 2 atomes de carbone, par le groupe aldéhyde ainsi que par le reste oxime et éther d'oxime.

3. Utilisation selon la revendication 1, caractérisée en ce qu'on utilise des dérivés substitués du tertiobutanol, de formule (I), dans laquelle

$R^1$ et $R^2$ sont identiques ou differénts et représentent chacun un groupe imidazol-1-yle, un groupe 1,2,4-triazol-1-yle, un groupe 1,2,4-triazol-4-yle ou un groupe pyrazol-1-yle, et

$R^3$ représente un groupe phénoxy, phénylthio, phénylamino ou phényl-N-alkyl-amino comportant 1 ou 2 atomes de carbone dans la partie alkyle, chacun de ces groupes étant éventuellement substitué une à trois fois, de façon identique ou différente, et l'on peut citer comme substituants fixés sur la partie phényle un atome de fluor, de chlore, de brome, un groupe méthyle, éthyle, isopropyle, tertiobutyle, 2-méthyl-but-2-yle, méthoxy, éthoxy, méthylthio, trifluorométhyle, trifluorométhoxy, trifluorométhylthio, cyclohexyle, nitro, cyano, hydroxycarbonyle, méthylcarbonyle, méthoxycarbonyle, éthoxycarbonyle, un groupe phényle ou phénoxy (chacun éventuellement substitué une à trois fois, de façon identique ou différente, par du chlore, par un groupe nitro, trifluorométhyle ou méthoxy), le groupe aldéhyde, le groupe hydroxyiminométhyle et le groupe méthoxyiminométhyle.

4. Utilisation de dérivés substitués du tertiobutanol de formule (I) selon la revendication 1 pour lutter contre des champignons afin de protéger les plantes.